# EUROPEAN PATENT APPLICATION

(11) **EP 4 729 935 A2**
(43) Date of publication of application: **22.04.2026**
(21) Application number: 26157422.2
(22) Date of filing: 20.12.2021
(51) Int. Cl.: G01N 27/623

(54) **AUTOMATED CLINICAL DIAGNOSTIC SYSTEM AND METHOD**

(30) Priority: 22.12.2020 EP 20216371
(62) Divisional of application: 21843660.8
(71) Applicant: F. Hoffmann-La Roche AG, 4070 Basel (CH); Roche Diagnostics GmbH, 68305 Mannheim (DE)
(72) Inventor: EPPING, Claudia, 82377 Penzberg (DE); REMPT, Martin, 82377 Penzberg (DE)
(74) Representative: Schach, Denise Kristine

(57) **Abstract**

The present invention relates to a clinical diagnostic system, a method for determining the presence or level of at least one analyte of interest in a biological sample, a kit and the uses thereof for efficiently and/or robust detection of an analyte of interest.

## Description

### Field of the Invention

The present invention relates to a clinical diagnostic system, a method for determining the presence or level of at least one analyte of interest in a biological sample, a kit and the uses thereof for efficiently and/or robust detection of an analyte of interest.

### Background of the Invention

Mass spectrometry (MS) is a widely used technique for the qualitative and quantitative analysis of chemical substances ranging from small molecules to macromolecules. In general, it is a very sensitive and specific method, allowing even for the analysis of complex biological, for example (e.g.), environmental or clinical samples. However, for several analytes, especially if analysed from complex biological matrices such as serum, sensitivity of the measurement remains an issue.

Often MS is combined with chromatographic techniques, particularly gas and liquid chromatography such as e.g. HPLC. Here, the analysed molecule (analyte) of interest is separated chromatographically and is individually subjected to mass spectrometric analysis (Higashi et al. (2016) J. of Pharmaceutical and Biomedical Analysis 130 p. 181-190). However, this system has inherent problems of analyte - surface contact and the need for stationary phases and liquid solvents. Furthermore the mechanical parts underlies an inherent stress by being moved and this results in the end in a deteriotion of the equipment. Therefore the systems need intensive maintenance due to the mechanical parts which are used. Stationary phases which are being used for e.g. GC or HPLC also need to be replaced by deteriotion or unremovable chemical blockage.

There is, however, still a need of replacing the HPLC of MS analysis methods in order to overcome the above mentioned disadvantages.

Additionally, mass spectrometry is a versatile tool over all disciplines in science. For environmental analyses, it can determine traces of pollutants, in mineralogy, it can assess chemical compositions and in chemistry, it can reflect pathways of reactions by measuring certain compounds qualitatively as well as quantitatively.

In clinical diagnostics, the versatile tool of mass spectrometry meets different aspects in terms of sample types (e.g. liquid (e.g. serum/blood), solid (e.g. cross sections of tissue) or gaseous (e.g. cross-sections of tissue)) as well as analytical tasks e.g. qualitative or quantitative.

Mass spectrometry in the clinical analysis is further hitting the needs for dealing with low mass analytes e.g. alkali metals (Na, K etc.) or small acids/bases e.g. valproic acid as well as medium size analytes (e.g. steroids) and large molecular mass analytes e.g. biopolymers like proteins. All of the analytes need to be addressed quantitatively as well as qualitatively.

Additionally, there is growing interest for the implementation of mass spectrometry in the clinical laboratory. The number of published methods especially for small molecules in therapeutic drug monitoring or drug of abuse testing is increasing.

Some ready to use kits for pre-validated clinical MS applications are becoming commercially available.

The use of mass spectrometry, however, even in connection with such kits, may not be regulatory approved for clinical diagnostics. This is mostly because of lack of standardized procedures, except for a very few analytes, and because of the still large number of user dependent factors, e.g. due to a number of manual steps that are still conducted and the diversity of hardware components that may be used and combined, and that play a role in delivering reliable and reproducible results of clinical relevance. In particular, sample preparation is typically a manual and tedious procedure. Protein precipitation with subsequent centrifugation is the most popular method to remove unwanted and potentially disturbing sample matrix. The use of kits may in part facilitate sample preparation that can be at least in part automated. Kits are however available only for a limited number of analytes of interest and the entire process from sample preparation, to separation and detection remains complex, requiring the attendance of highly trained laboratory personnel to run highly sophisticated instruments.

Also, typically, a batch approach is followed, where a batch of samples prepared in advance under the same preparation conditions undergo consecutive separation runs under the same separation conditions. This approach however does not enable high throughput and is not flexible, e.g. does not allow re-scheduling (changing a pre-defined processing sequence) in view for example of incoming emergency samples that have higher priority and have to be processed first.

A system and a method are herein described that make use mass spectrometry more convenient and more reliable and therefore suitable for clinical diagnostics. In particular, high-throughput, e.g. up to 100 samples/hour or more with random access for analytes and and sample concentrations/ matrix components sample preparation can be obtained. Moreover the process can be fully automated increasing the walk-away time and decreasing the level of skills required.

There is thus an urgent need in the art for a clinical diagnostic system and a method which allows for a compfortable, low maintainance and fast detection of analytes from complex biological matrices.

Further, the coupling of a sample containing an analyte of interest as decribes herein via an ion generation source to an ion mobility unit, e.g. a structures for lossless ion manipulation unit and an ion detection system can overcome the above-mentioned limitations.

It is an object of the present invention to provide a clinical diagnostic system, a method for determining the presence or level of at least one analyte of interest in a biological sample, a kit and/or the uses thereof for efficiently and/or low maintenance and/or fast and/or robust detection of an analyte of interest.

This object is or these objects are solved by the subject matter of the independent claims. Further embodiments are subjected to the dependent claims.

### Summary of the Invention

In the following, the present invention relates to the following aspects:
In a first aspect, the present invention relates to a clinical diagnostic system comprising
- a sample preparation station for the automated preparation of biological samples comprising at least one analyte of interest,
- an ion generation station for generating at least one ion or ions of the at least one analyte of interest,
- at least one selectivity enhancer station or more for enhancing the selectivity of the at least one analyte of interest, wherein the at least one selectivity enhancer station separates ions based on the respective ion size comprises an ion mobility unit for separation ions with respect to their ion mobility using electric and/or radio frequency fields (AC/DC and/or RF) applied to one or more electrodes,
- an ion detection station for detecting of at least one ion of the at least one analyte of interest, and
- a data processing station for processing and/or evaluation of at least one electronic signal received at least from the ion detection system,
preferably wherein the clinical diagnostic system is free of a chromatography unit or station.

In a second aspect, the present invention relates to the use of the clinical diagnostic system of the first aspect of the present invention for determining the presence or the level of the at least one analyte of interest in the biological sample.

In a third aspect, the present invention relates to a method for determining the presence or level of at least one analyte of interest in a biological sample comprising:
A) Preparation of the biological sample, preferably automated preparation of the biological sample comprising at least one analyte of interest,
B) Ion generation from the at least one analyte of interest,
C) Separation the ion or ions via their ion mobility using an electric field applied to one or more electrodes,
D) Detection and determining of at least one ion of the at least one analyte of interest using mass spectrometry.

In a fourth aspect, the present invention relates to a kit suitable to perform a method of the third aspect of the invention comprising
- reagents, preferably for performing the pre-treatment,
- magnetic, paramagnetic or supraparamagnetic beads for performing the enrichment, and
- optionally an internal standard, which is preferably isotopically labelled.

In a fifth aspect, the present invention relates to the use of a kit in a clinical diagnostic system of the first aspect of the invention and/or in a method of third aspect of the invention.

### Description of the Figures

**Figures 1 to 8** show a schematic representation of a clinical diagnostic system and a clinical diagnostic method.
**Figure 9** shows schematically elements of a clinical diagnostic method and in particular a combination of possible workflow paths, depending on the sample type and/or the analytes of interest in a sample.
**Figures 10** **and** **11** show an examplified flow chart of the clinical diagnostic method.
**Figure 12A** **and** **12B** show two generic examples of analyte specific workflow paths that may be pre-defined in a master table or memory, each including a selection of options among generally selectable options.
**Figure 13** shows the separation of three isobaric pentasaccharides using SLIM-MS according to a comparative example.

### Detailed Description of the Invention

Before the present invention is described in detail below, it is to be understood that this invention is not limited to the particular embodiments and examples described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Several documents are cited throughout the text of this specification. Each of the documents cited herein (including all patents, patent applications, scientific publications, manufacturer's specifications, instructions etc.), whether supra or infra, is hereby incorporated by reference in its entirety. In the event of a conflict between the definitions or teachings of such incorporated references and definitions or teachings recited in the present specification, the text of the present specification takes precedence.

In the following, the elements of the present invention will be described. These elements are listed with specific embodiments, however, it should be understood that they may be combined in any manner and in any number to create additional embodiments. The various described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

The word **"comprise",** and variations such as "comprises" and "comprising", will be understood to imply the inclusion of a stated integer or step or group of integers or steps but not the exclusion of any other integer or step or group of integers or steps. The term "including" and "comprising" can be used interchangeable.

As used in this specification and the appended claims, the singular forms **"a", "an",** and **"the"** include plural referents, unless the content clearly dictates otherwise.

**Percentages, concentrations, amounts, and other numerical data** may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "4% to 20 %" should be interpreted to include not only the explicitly recited values of 4 % to 20 %, but to also include individual values and sub-ranges within the indicated range. Thus, included in this numerical range are individual values such as 4, 5, 6, 7, 8, 9, 10, ... 18, 19, 20 % and sub-ranges such as from 4-10 %, 5-15 %, 10-20%, etc. This same principle applies to ranges reciting minimal or maximal values. Furthermore, such an interpretation should apply regardless of the breadth of the range or the characteristics being described.

The term **"about"** when used in connection with a numerical value is meant to encompass numerical values within a range having a lower limit that is 5% smaller than the indicated numerical value and having an upper limit that is 5% larger than the indicated numerical value.

A **"clinical diagnostics system"** is preferably a laboratory automated apparatus dedicated to the analysis of samples for in vitro diagnostics. The clinical diagnostics system may have different configurations according to the need and/or according to the desired laboratory workflow. Additional configurations may be obtained by coupling a plurality of stations and/or apparatuses and/or modules and/or units together. A "unit" is a work cell, typically smaller in size than the entire clinical diagnostics system, which has a dedicated function. This function can be analytical but can be also pre-analytical or post analytical or it can be an auxiliary function to any of the pre-analytical function, analytical function or post-analytical function. In particular, a unit can be configured to cooperate with one or more other modules or units for carrying out dedicated tasks of a sample processing workflow, e.g. by performing one or more pre-analytical and/or analytical and/or post-analytical steps. The term "unit" and device can be used interchangeably. In particular, the clinical diagnostics system can comprise one or more analytical apparatuses, designed to execute respective workflows that are optimized for certain types of analysis, e.g. clinical chemistry, immunochemistry, coagulation, hematology, liquid chromatography separation, mass spectrometry, etc. Thus the clinical diagnostic system may comprise one analytical apparatus or a combination of any of such analytical apparatuses with respective workflows. Each of the analytical apparatuses may comprise at least one station and/or at least one unit, preferably apre-analytical and/or post analytical unit. The clinical diagnostics system can comprise functional units such as liquid handling units for pipetting and/or pumping and/or mixing of samples and/or reagents and/or system fluids, and also functional units for sorting, storing, transporting, identifying, separating, detecting.

The term **"determining"** the level of the analyte of interest, as used herein refers to the quantification or qualification of the analyte of interest, e.g. to determining or measuring the level of the analyte of interest in the pretreated sample.

In this context **"presence of at least one analyte of interest"** encompass the qualification of the analyte of interest by its absolute value and/or its relative signal value to a internal standard/ other analyte and/ or a limit of analyte concentration which is matched with its conentration.

In this context **"level"** or **"level value"** encompasses the absolute amount, the relative amount or concentration as well as any value or parameter which correlates thereto or can be derived therefrom.

In the context of the present disclosure, the term **"analyte",** "analyte molecule" , or "analyte(s) of interest" are used interchangeably referring the chemical species to be analysed via mass spectrometry. Chemical species suitable to be analysed via mass spectrometry, i.e. analytes, can be any kind of molecule present in a living organism, include but are not limited to nucleic acid (e.g. DNA, mRNA, miRNA, rRNA etc.), amino acids, peptides, proteins (e.g. cell surface receptor, cytosolic protein etc.), metabolite or hormones (e.g. testosterone, estrogen, estradiol, etc.), fatty acids, lipids, carbohydrates, steroids, ketosteroids, secosteroids (e.g. Vitamin D), molecules characteristic of a certain modification of another molecule (e.g. sugar moieties or phosphoryl residues on proteins, methyl-residues on genomic DNA) or a substance that has been internalized by the organism (e.g. therapeutic drugs, drugs of abuse, toxins, etc.) or a metabolite of such a substance. Such analyte may serve as a biomarker. In the context of present invention, the term "biomarker" refers to a substance within a biological system that is used as an indicator of a biological state of said system.

The term **"are connected to each other",** as used herein can refer or refers to a physical contact by either a stationary unit e.g. tube connection or a semi physical connection by. e.g. a pipetting unit. Furthermore physical can mean a contact in solid/liquid (e.g. aqustic droplet ejection or piezo driven droplet ejection) or gaseous (e.g. ion optic with an ion mirror, an S-lense or a multipole).

The term **"are connected to each other",** as used herein can refer or refers to an electrical contact and/or electromagnetic contact between stations and/or units.

The term **"directly",** as used herein may mean that no other stations are arranged between the two directly connected stations.

Analytes may be present in a sample of interest, e.g. a biological sample, preferably a clinical biological sample. The term **"sample"** or "sample of interest" or "biological sample" are used interchangeably herein, referring to a part or piece of a tissue, organ or individual, typically being smaller than such tissue, organ or individual, intended to represent the whole of the tissue, organ or individual. Upon analysis a sample provides information about the tissue status or the health or diseased status of an organ or individual. Examples of samples include but are not limited to fluid samples such as blood, serum, plasma, synovial fluid, spinal fluid, urine, saliva, and lymphatic fluid, or solid samples such as dried blood spots and tissue extracts. Further examples of samples are cell cultures or tissue cultures.

The term **"serum"** as used herein is the clear liquid part of the blood hat can be separated from clotted blood. The term **"plasma"** as used herein is the clear liquid part of blood which contains the blood cells. Serum differs from plasma, the liquid portion of normal unclotted blood containing the red and white cells and platelets. It is the clot that makes the difference between serum and plasma. The term **"whole blood"** as used herein contains all components of blood, for examples white and red blood cells, platelets, and plasma.

In the context of the present disclosure, the sample may be derived from an **"individual"** or "subject" or "patient". Typically, the subject or patient is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the patient smaple is from a human.

The term **"small molecule"** as used herein is a molecule or compound having a molecular weight or molar mass of less than 2000 Da preferably less than 1000 Da, more preferably 800 Da.

A **"sample preparation station"** is a pre-analytical module coupled to one or more analytical stations or units designed to execute a series of sample processing steps aimed at removing or at least reducing interfering matrix components in a sample and/or enriching analytes of interest in a sample. Such processing steps may include any one or more of the following processing operations carried out on a sample or a plurality of samples, sequentially, in parallel or in a staggered manner: pipetting (aspirating and/or dispensing) fluids, pumping fluids, mixing with reagents, incubating at a certain temperature, heating or cooling, centrifuging, separating, filtering, sieving, drying, washing, resuspending, aliquoting, transferring, storing...).

A **"reagent"** is a substance used for treatment of a sample in order e.g. to prepare a sample for analysis, to enable a reaction to occur, or to enable detection of a physical parameter of the sample or analyte contained in the sample. In particular, a reagent can be a substance that is or comprises a reactant, typically a compound or agent capable e.g. of binding to or chemically transforming one or more analytes present in a sample or an unwanted matrix component of the sample. Examples of reactants are enzymes, enzyme substrates, conjugated dyes, protein-binding molecules, ligands, nucleic acid binding molecules, antibodies, chelating agents, promoters, inhibitors, epitopes, antigens, and the like. However the term reagent is used to include any fluid that can be added to a sample including a dilution liquid, including water or other solvent or a buffer solution, or a substance that is used for disruption of specific or nonspecific binding of an analyte to a protein, binding proteins or surfaces.

Sample may be provided for example in sample containers such as sample tubes, including primary tubes and secondary tubes, or multi-well plates, or any other sample carrying support. Reagents may be arranged for example in the form of containers or cassettes containing individual reagents or group of reagents and placed in appropriate receptacles or positions within a storage compartment or conveyor. Other types of reagents or system fluids may be provided in bulk containers or via a line supply.

The term **"automatically"** or **"automated"** as used herein is a broad term and is to be given its ordinary and customary meaning to a person of ordinary skill in the art and is not to be limited to a special or customized meaning. The term specifically may refer, without limitation, to a process which is performed completely by means of at least one computer and/or computer network and/or machine, in particular without manual action and/or interaction with a user.

The term **"fully automated"** may refer to a process which is performed completely by means of at least one computer and/or computer network and/or machine, without manual action and/or interaction with a user.

The term **"partially automated"** may refer to a process which is performed by means of at least one computer and/or computer network and/or machine and with the aid of manual action and/or interaction with a user. Preferably, "partially automated" can mean that the manual action and/or interaction with a user is 50% or 40% or 30% or 20% or 10% or 5% at the maximum of the total process, wherein the rest of the process is performed by means of at least one computer and/or computer network and/or machine. "By means of at least one computer and/or computer network and/or machine" can mean that this process is performed without any manual action and/or interaction with a user.

Typically, an **"internal standard"** (ISTD) is a known amount of a substance which exhibits similar properties as the analyte of interest when subjected to the mass spectrometric detection workflow (i.e. including any pre-treatment, enrichment and actual detection step). Although the ISTD exhibits similar properties as the analyte of interest, it is still clearly distinguishable from the analyte of interest. Exemplified, during ion mobility separation, the ISTD has the same ion size but different m/z ratio than the analyte of interest. Preferably the internal standard is in its ion size not distinguishable from the analyte but different in the m/z ratio. Thus, both the analyte and the ISTD enter the mass spectrometer at the same time. The ISTD however, exhibits a different molecular mass than the analyte of interest from the sample. This allows a mass spectrometric distinction between ions from the ISTD and ions from the analyte by means of their different mass/charge (m/z) ratios. Both are subject to fragmentation and provide daughter ions. These daughter ions can be distinguished by means of their m/z ratios from each other and from the respective parent ions. Consequently, a separate determination and quantification of the signals from the ISTD and the analyte can be performed. Since the ISTD has been added in known amounts, the signal intensity of the analyte from the sample can be attributed to a specific quantitative amount of the analyte. Thus, the addition of an ISTD allows for a relative comparison of the amount of analyte detected, and enables unambiguous identification and quantification of the analyte(s) of interest present in the sample when the analyte(s) reach the mass spectrometer. Typically, but not necessarily, the ISTD is an isotopically labeled variant (comprising e.g. ²H, ¹³C, or ¹⁵N etc. label) of the analyte of interest.

In the context of the present invention, the term **"derivatisation reagent"** or **"label"** or **"derivatisation agent"** are used interchangeably and refer to a chemical substance having a specific chemical structure. Said compound may comprise one or more reactive groups. Each reactive group may fulfil a different functionality, or two or more reactive groups may fulfil the same funtion. Reactive groups include but are not limited to reactive units, charged units, and neutral loss units. The derivatization reagent can undergo an atmospheric pressure chemical reaction with the analyte of interest.

The term **"hemolysis reagent"** (HR) refers to reagents which lyse cells present in a sample, in the context of this invention hemolysis reagents in particular refer to reagents which lyse the cell present in a blood sample including but not limited to the erythrocytes present in whole blood samples. A well known hemolysis reagent is water (H₂O). Further examples of hemolysis reagents include but are not limited to deionized water, liquids with high osmolarity (e.g. 8M urea), ionic liquids, and different detergents.

In the context of the present invention, the term **"compound"** or **"derivatisation reagent"** or **"label"** or **"derivatisation agent"** are used interchangeably and refer to a chemical substance having a specific chemical structure. Said compound may comprise one or more reactive groups Q. Each reactive group may fulfil a different functionality, or two or more reactive groups may fulfil the same funtion. Reactive groups include but are not limited to reactive units, charged units, and neutral loss units. The derivatization reagent can undergo a chemical reaction with the analyte of interest.

The term **"solid-phase extraction (SPE)"** refers to the adsorption of analytes from the a matrix, e.g. a biological matrix onto a solid sorbent, and subsequent elution of the analytes from the sorbent into an solvent, e.g. an organic solvent. SPE is an efficient tool for the sample preparation in chemistry. Conventional SPE materials are silica-based, carbon-based, and clay-based resins. Many adsorbents for SPE applications are commercially available in different formats in the market such as SPE tubes and pipette tip formats such as Oasis-HLB (produced by Waters), Omix (produced by Agilent), and MonoTips (produced by GL Sciences).

The basic principles of SPE are similar to liquid-liquid extraction. Both methods involve the distribution of dissolved species between two phases. However, SPE involves the dispersion of the analyte between a liquid (sample medium) and a solid (adsorbent) phase instead of the two liquid phases which cannot be not mixed together as in liquid-liquid extraction. This technique allows the enrichment and purification of the analytes on a solid adsorbent through adsorption from the solution. This technique is known for a skilled person and thus not explained in more detail.

The term **"liquid-liquid extraction (LLE)"** refers to a separation process consisting of the transfer of a solute from one solvent to another, the two solvents being immiscible or partially miscible with each other. Frequently, one of the solvents can be water or an aqueous mixture and the other can be a nonpolar organic liquid. As in all extraction processes, liquid-liquid extraction comprises a step of mixing (contacting), followed by a step of phase separation. It is important to consider both steps in the selection of solvents and modes of operation. Thus, while vigorous mixing is favorable to the transfer of the analyte of interest from one solvent to the other, it may also impair the ease of phase separation by forming emulsions. This technique is known for a skilled person and thus not explained in detail.

The term **"chromatography"** refers to a process in which a chemical mixture carried by a liquid or gas is separated into components as a result of differential distribution of the chemical entities as they flow around or over a stationary liquid or solid phase.

The term **"liquid chromatography"** or "LC" refers to a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (i.e., mobile phase), as this fluid moves relative to the stationary phase(s). Methods in which the stationary phase is more polar than the mobile phase (e.g., toluene as the mobile phase, silica as the stationary phase) are termed normal phase liquid chromatography (NPLC) and methods in which the stationary phase is less polar than the mobile phase (e.g., water-methanol mixture as the mobile phase and C18 (octadecylsilyl) as the stationary phase) is termed reversed phase liquid chromatography (RPLC).

**"High performance liquid chromatography"** or "HPLC" refers to a method of liquid chromatography in which the degree of separation is increased by forcing the mobile phase under pressure through a stationary phase, typically a densely packed column. Typically, the column is packed with a stationary phase composed of irregularly or spherically shaped particles, a porous monolithic layer, or a porous membrane. HPLC is historically divided into two different sub-classes based on the polarity of the mobile and stationary phases. Methods in which the stationary phase is more polar than the mobile phase (e.g., toluene as the mobile phase, silica as the stationary phase) are termed normal phase liquid chromatography (NPLC) and the opposite (e.g., water-methanol mixture as the mobile phase and C18 (octadecylsilyl) as the stationary phase) is termed reversed phase liquid chromatography (RPLC). Micro LC refers to a HPLC method using a column having a norrow inner column diameter, typically below 1 mm, e.g. about 0.5 mm. "Ultra high performance liquid chromatography" or "UHPLC" refers to a HPLC method using a pressure of 120 MPa (17,405 lbf/in2), or about 1200 atmospheres. Rapid LC refers to an LC method using a column having an inner diameter as mentioned above, with a short length <2 cm, e.g. 1 cm, applying a flow rate as mentioned above and with a pressure as mentioned above (Micro LC, UHPLC). The short Rapid LC protocol includes a trapping / wash / elution step using a single analytical column and realizes LC in a very short time <1 min.

Further well-known LC modi include "hydrophilic interaction chromatography" (HILIC), size-exclusion LC, ion exchange LC, and affinity LC.

LC separation may be single-channel LC or multi-channel LC comprising a plurality of LC channels arranged in parallel. In LC analytes may be separated according to their polarity or log P value, size or affinity, as generally known to the skilled person.

The term **"micro particle"** may refer to spheric particles or unregularly shaped particles in a range of 10 nm-10 mm. The particles can consist of or comprise inorganic and-or organic material e.g. SiO2, TiO2, Polystyrene, PMMA and optionally many more materials. The surface of the particles can be modified by a different chemistry from the supporting microparticle core material e.g. C18 chain, CN, Pentaflurophenyl etc.

The term **"bead"** does not necessarily refer to a spherical shape but to a particle having an average size in the nanometer or micrometer range and having any possible shape. A bead may be a solid phase. Suitable solid phases include but are not limited to Solid Phase Extraction (SPE) cartridges. Beads may be non-magnetic, magnetic, or paramagnetic. Beads may be coated differently to be specific for the analyte of interest. The coating may differ depending on the use intended, i.e. on the intended capture molecule. It is well-known to the skilled person which coating is suitable for which analyte. The beads may be made of various different materials. The beads may have various sizes and comprise a surface with or without pores.

Non-magnetic beads may also be used. In that case capturing and releasing may be based on filtration. The sample preparation station may further comprise one or more pipetting device or fluid transport device for adding/removing fluids, such as samples, reagents, wash fluids, suspension fluids, into/from the reaction container(s).

The term **"inert gas stripping"** may refer to the process of bubbeling an inert gas e.g. nitrogen or argon through a liquid which needs to be stripped. Volatile compounds gets evaporated due to the equilibrium process of the inert gas with the analyte supported liquid.

The term **"headspace extraction"** may refer to the process of having a liquid sample seald gastight in a vial which has air or other gases in the headspace over the liquid. The sealing of the vial is at ambigent pressure. The seald vial is getting heated by external heat source and the analyte of interest evaporats in the headspace while increasing the pressure of the vial. The gaseous portion of the vial if further taken of by a suringe or capillary and directly transfered to the analytical unit.

The term **"gas adsorption supported by liquid and/or solid"** may refer to a process in which a gas is adsorb on the active surface of a liquid or a solid. The analyte can undergo during this process endothermal or exothermal processes as well as chemical reactions.

The term **"unsteady analyte ion supply unit"** may refer to a unit which produces ions time dependend e.g. by a laser shot which is pulsed.

The term **"steady analyte ion supply unit"** may refer to a unit which produces ions time independend e.g. by a constant low and power supply during nano-ESI.

The term **"electrospray ionization"** or "ESI," refers to methods in which a solution is passed along a short length of capillary tube, to the end of which is applied a high positive or negative electric potential. Solution reaching the end of the tube is vaporized (nebulized) into a jet or spray of very small droplets of solution in solvent vapor. This mist of droplets flows through an evaporation chamber, which is heated slightly to prevent condensation and to evaporate solvent. As the droplets get smaller the electrical surface charge density increases until such time that the natural repulsion between like charges causes ions as well as neutral molecules to be released.

The term **"nano electrospray ionization"** or **"nanoESI"** refers to methods typically using flow rates below 1 µL/min either in static or dynamic mode. Preferably, nanoESI uses a flow rate of 50 to 500 nl/min, e.g. 500 nl/min. 500 nl/min is equal to 0.5 µl/min.

The term **"static nanoESI"** is used in the context of the present disclosure as a non-continuous flow nanoESI option. The analysis is typically defined by a discrete sample being loaded by single-use pipette tips into an emitter. In contrast, dynamic nanoESI mass spectrometry is characterized by a mobile phase pumped at low flow rates through a small diameter emitter.

The term **"atmospheric pressure chemical ionization"** or "APCI," refers to mass spectrometry methods that are similar to ESI; however, APCI produces ions by ion-molecule reactions that occur within a plasma at atmospheric pressure. The plasma is maintained by an electric discharge between the spray capillary and a counter electrode. Then ions are typically extracted into the mass analyser by use of a set of differentially pumped skimmer stages. A counterflow of dry and preheated nitrogen gas may be used to improve removal of solvent. The gas-phase ionization in APCI can be more effective than ESI for analysing less-polar entity.

**"High-field asymmetric-waveform ion-mobility spectrometry (FAIMS)"** is an atmospheric pressure ion mobility technique that separates gas-phase ions by their behavior in strong and weak electric fields.

The term "Nano Spray" or "APCI," refers to two independend ionization techniques in which a high electrical current is being used for the ionization.

The term "is arranged before" can refer to the arrangement of the two units, which means that the at least one analyte of interest passes at first the first unit (e.g. first ion fragmentation), which is arranged before the second unit (e.g. ion mobility unit), and then passes the second unit.

The term "is arranged after" can refer to the arrangement of the two units, that two units are arranged after one another. This can mean that the at least one analyte of interest passes at first the first unit (e.g. ion mobility unit) and then the second unit (e.g. second ion fragmentation), which is arranged after the first unit.

The term **"Mass Spectrometry"** ("Mass Spec" or "MS") or "mass spectrometric determination" relates to an analytical technology used to identify compounds by their mass. MS is a methods of filtering, detecting, and measuring ions based on their mass-to-charge ratio, or "m/z". MS technology generally includes (1) ionizing the compounds to form charged compounds; and (2) detecting the molecular weight of the charged compounds and calculating a mass-to-charge ratio. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). The term "ionization" or "ionizing" refers to the process of generating an analyte ion having a net electrical charge equal to one or more electron units. Negative ions are those having a net negative charge of one or more electron units, while positive ions are those having a net positive charge of one or more electron units. The MS method may be performed either in "negative ion mode", wherein negative ions are generated and detected, or in "positive ion mode" wherein positive ions are generated and detected.

**"Tandem mass spectrometry"** or "MS/MS" involves multiple steps of mass spectrometry selection, wherein fragmentation of the analyte occurrs in between the stages. In a tandem mass spectrometer, ions are formed in the ion source and separated by mass-to-charge ratio in the first stage of mass spectrometry (MS1). Ions of a particular mass-to-charge ratio (precursor ions or parent ion) are selected and fragment ions (or daughter ions) are created by collision-induced dissociation, ion-molecule reaction, or photodissociation. The resulting ions are then separated and detected in a second stage of mass spectrometry (MS2). Since a mass spectrometer separates and detects ions of slightly different masses, it easily distinguishes different isotopes of a given element. Mass spectrometry is thus, an important method for the accurate mass determination and characterization of analytes, including but not limited to low-molecular weight analytes, peptides, polypeptides or proteins. Its applications include the identification of proteins and their post-translational modifications, the elucidation of protein complexes, their subunits and functional interactions, as well as the global measurement of proteins in proteomics. De novo sequencing of peptides or proteins by mass spectrometry can typically be performed without prior knowledge of the amino acid sequence.

**"Multiple reaction mode"** or "MRM" is a detection mode for a MS instrument in which a precursor ion and one or more fragment ions are selectively detected.

The term **"in vitro method"** is used to indicate that the method is performed outside a living organism and preferably on body fluids, isolated tissues, organs or cells.

The term **"random-excess mode"** or **"random-excess"** as used herein is a broad term and is to be given its ordinary and customary meaning to a person of ordinary skill in the art and is not to be limited to a special or customized meaning. The term specifically may refer, without limitation, to a process that does not detect and/or measure at least one ion of the at least one analyte of interest sequentially, which can mean starting from the beginning and proceeding step by step. Random access or direct access may refer to the ability to access an arbitrary analyte of interest and/or sample of a sequence in equal time or any date from a population of addressable analyte of interest and/or sample roughly as easily and efficiently as any other, no matter how many analytes of interest and/or samples may be in the set.

A **"kit"** is any manufacture (e.g., a package or container) comprising at least one reagent, e.g., a medicament for treatment of a disorder, or a probe for specifically detecting a biomarker gene or protein of the invention. The kit is preferably promoted, distributed, or sold as a unit for performing the methods of the present invention. Typically, a kit may further comprise carrier means being compartmentalized to receive in close confinement one or more container means such as vials, tubes, and the like. In particular, each of the container means comprises one of the separate elements to be used in the method of the first aspect. Kits may further comprise one or more other reagents including but not limited to reaction catalyst. Kits may further comprise one or more other containers comprising further materials including but not limited to buffers, diluents, filters, needles, syringes, and package inserts with instructions for use. A label may be present on the container to indicate that the composition is used for a specific application, and may also indicate directions for either in vivo or in vitro use. The computer program code may be provided on a data storage medium or device such as a optical storage medium (e.g., a Compact Disc) or directly on a computer or data processing device. Moreover, the kit may, comprise standard amounts for the analytes of interest for calibration purposes.

### Embodiments

In a first aspect, the present invention relates to a clinical diagnostic system comprising
- a sample preparation station for the automated preparation of biological samples comprising at least one analyte of interest,
- an ion generation station for generating at least one ion or ions of the at least one analyte of interest,
- at least one selectivity enhancer station or more for enhancing the selectivity of the at least one analyte of interest, wherein the at least one selectivity enhancer station separates ions based on the respective ion size comprises an ion mobility unit for separation ions with respect to their ion mobility using electric and/or radio frequency fields (AC/DC and/or RF) applied to one or more electrodes,
- an ion detection station for detecting of at least one ion of the at least one analyte of interest, and
- a data processing station for processing and/or evaluation of at least one electronic signal received at least from the ion detection system.

In embodiments of the first aspect of the invention, the clinical diagnostic system is for determining the presence or the level of at least one analyte of interest.

In embodiments of the first aspect of the invention, the analyte of interest is artificial or a native.

In embodiments of the first aspect of the invention, clinical diagnostic system is free of a chromatography unit or station, preferably free of a liquid chromatography (LC) unit and/or high pressure liquid chromatography (HPLC) unit and/or gas chromatography (GC) unit.

In embodiments of the first aspect of the invention, the sample preparation station, the ion generation station, the at least one selectivity enhancer station, the ion detection station and the data processing station are connected to each other. Preferably, the sample preparation station is directly connected to the ion generation station, which is directly connected to the at least one selectivity enhancer station, which is directly connected to the ion detection station.

In embodiments of the first aspect of the invention, the biological sample are prepared in a random-excess mode. The meaning of a random-excess mode is known for a skilled person and thus not explained in detail. Additionally or alternatively, the the clinical diagnostic system is automated, preferably fully or partially automated.

In embodiments of the first aspect of the invention, the biological sample is obtained from a patient sample, which is selected from a group consisting of serum, plasma, tissue, liquor, breath, sweat, sputum and whole blood sample from an individual.

In embodiments of the first aspect of the invention, the individual is a mammal, preferably a human.

In embodiments of the first aspect of the invention, the at least one analyte of interest is a small molecule.

In embodiments of the first aspect of the invention, the at least one analyte of interest has a molar mass of smaller than m/z (mass-to-charge ratio) = 2000, preferably smaller than m/z =1000 or smaller than m/z =900 or smaller than m/z =800 or smaller than m/z =700 or smaller than m/z =600 or smaller than m/z =500 or smaller than m/z =400 or smaller than m/z =300 or smaller than m/z =200 or smaller than m/z =100 or smaller than m/z =90.

In embodiments of the first aspect of the invention, the at least one analyte of interest is not a large molecule having a m/z ratio of < 2000, preferably the at least one analyte of interest is selected from the following group: testosterone, vitamin D, cyclosporine A, lidocaine, sirolimus, abeta 42.

In embodiments of the first aspect of the invention, the at least one analyte of interest is selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.

In embodiments of the first aspect of the invention, the biological samples comprises the at least one analyte of interest and a matrix, wherein preferably the matrix comprises the following components or combinations thereof: inorganic salt, organic salt component, protein, solvents polymer, small molecule.

An inorganic salt is e.g. (for example) sodium chloride.

An organic salt component is e.g. HEPES ((4-(2-hydroxyethyl)-1-piperazineethanesulfonic acid) or citric acid.

A protein is e.g. human serum albumin.

A solvent is e.g. water, methanol and/or alcohol.

A polymer is e.g. DNA and/or polyacrylamide.

A small molecule is e.g. formaldehyde.

In embodiments of the first aspect of the invention, the matrix is separated from the at least one analyte of interest in the sample preparation station.

The clinical diagnostic system comprises a sample preparation station. The sample preparation station is for the automated preparation of biological samples. The at least one biological sample comprises at least one analyte of interest.

In embodiments of the first aspect of the invention, the sample preparation station comprises at least one of the following units or combinations thereof: sample delivery unit, sample pre-treatment unit, sample analytical unit.

In embodiments of the first aspect of the invention, the sample preparation station is partially or fully automated.

In embodiments of the first aspect of the invention, the sample preparation station is for removing or at least reducing interfering matrix components in the biological sample and/or enriching analytes of interest in the biological sample. Additionally, the sample preparation station can comprise at least one of the following units or combinations thereof: sample delivery unit, sample pre-treatment unit, sample analytical unit

In embodiments of the first aspect of the invention, the sample delivery unit comprises sample collection and/or preparation and/or transportation, e.g. including sample data preparation. This unit can consist of or comprises pipettors, gripper, conveyor and liquid handling robotors.

In the first aspect of the invention, the sample pre-treatment unit comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit.

In embodiments of the first aspect of the invention, the magnetic bead handling unit for treating samples with reagents comprising magnetic beads carrying analyte and/or matrix selective groups for extracting/enriching analytes of interest and removing or at least reducing matrix components. In particular, the magnetic bead handling unit comprises at least one magnetic or electromagnetic workstation for holding at least one reaction container and for manipulating magnetic beads added to a sample or samples contained therein. The magnetic bead handling unit may further comprise a mixing mechanism for mixing fluids and/or resuspending the magnetic beads in the reaction container(s), e.g. by shacking or agitating the reaction container(s), e.g. by an eccentric rotation mechanism. Alternatively the bead handling unit may be a flow-through system where the magnetic beads are captured in a channel or capillary flow-through device. According to this embodiment, capturing, washing and releasing of analytes can be done by repeatedly magnetically capturing and releasing beads in a flow-through channel.

In embodiments of the first aspect of the present invention, the magnetic bead handling unit is for treating the biological sample with magnetic beads carrying analyte and/or matrix selective groups.

In embodiments of the first aspect of the present invention, magnetic or paramagnetic beads carrying analyte-selective groups are added in the magnetic bead handling unit to the sample. In embodiments of the first aspect of the present invention, the addition of the magnetic beads comprises agitation or mixing. A pre-defined incubation period for capturing the analyte(s) of interest on the bead follows. In embodiments of the first aspect of the present invention, the workflow, which takes place in the magnetic bead handling unit, comprises a washing step (W1) after incubation with the magnetic beads. Depending on the analyte(s) one or more additional washing steps (W2) are performed. One washing step (W1, W2) comprises a series of steps including magnetic bead separation by a magnetic bead handling unit comprising magnets or electromagnets, aspiration of liquid, addition of a washing buffer, resuspension of the magnetic beads, another magnetic bead separation step and another aspiration of the liquid. Moreover washing steps may differ in terms of type of solvent (water/organic/salt/pH), apart from volume and number or combination of washing cycles. It is well-known to the skilled person how to choose the respective parameters. The last washing step (W1, W2) is followed by the addition of an elution reagent followed by resuspension of the magnetic beads and a pre-defined incubation period for releasing the analyte(s) of interest from the magnetic beads. The bound-free magnetic beads are then separated and the supernatant containing derivatized analyte(s) of interest is captured.

In embodiments of the first aspect of the present invention, the magnetic beads carrying matrix-selective groups are added in the magnetic bead handling unit to the sample. In embodiments of the first aspect of the present invention, the addition of the magnetic beads comprises agitation or mixing. A pre-defined incubation period for capturing the matrix on the bead follows. Here, the analyte of interest does not bind to the magnetic beads but remains in the supernatant. Thereafter, the magnetic beads are separated and the supernatant containing the enriched analyte(s) of interest is collected.

In embodiments of the first aspect of the present invention, the supernatant is subjected to enrichment handling unit performing e.g. a second enrichment workflow. Here, the supernatant is transferred to the at least one selectivity enhancer station or is transferred to the at least one selectivity enhancer station after a dilution step by addition of a dilution liquid. Different elution procedures/reagents may also be used, by changing e.g. the type of solvents (water/organic/salt/pH/micoparticles) and volume. The various parameters are well-known to the skilled person and easily chosen.

In alternative or in addition to magnetic bead handling unit, other techniques and/or units may be used such as protein precipitation followed by centrifugation, cartridge based solid phase extraction, pipette tip based solid phase extraction, liquid liquid extraction, affinity based extraction (immunosorption, molecular imprints, aptamers, etc).

In embodiments of the first aspect of the present invention, the magnetic bead handling unit is arranged after the sample delivery unit and before the sample analytical unit.

In embodiments of the first aspect of the invention, the internal standard handling unit consist of a reservoir in which the internal standard with a defined concentration for the respective analyte is stored. From this reservoir a dedicated proportion is taken out and givin to the native/ or prepared sample (liquid to liquid or gasous to gasous).

In embodiments of the first aspect of the present invention, the internal standard handling unit is for the addition of at least one internal standard and/or standard addition.

In embodiments of the first aspect of the present invention, internal standard handling unit is arranged after the sample delivery unit and before the sample analytical unit.

In embodiments of the first aspect of the invention, the supporting compound handling unit is for the addition of supporting compounds to release the analyte from a respective binder. The binder may be Vitamin-D binding protein or albumin for Vitamin D or linoleic acid as analyte of interest.

In embodiments of the first aspect of the invention, supporting compounds are selected from the following group: methanol, NaOH, Ethylenecarbonat, trifluoroacetic acid (TFA), formic acid, bovine serum albumin (BSA), Ammoniumfluoride.

In embodiments of the first aspect of the invention, the supporting compound handling unit is for the addition of supporting compounds or ion supporting molecules, e.g. in order to release the at least one analyte of interest from a binder, e.g. methanol or NaOH, or from a matrix or in order to add a matrix, e.g. 2,5-Dihydroxybenzoesäure (DHB) for MALDI applications or treat the analyte with a respective derivatization agent to change the chemical and/or physical properties of the analyte and/or the matrix.

In embodiments of the first aspect of the present invention, the supporting compound handling unit is arranged after the sample delivery unit and before the sample analytical unit.

In embodiments of the first aspect of the invention, the pipetting unit can consit of or comprises a flow through needle or a direct dispensing unit to take liquid from one place to another with a defined volume. The pipetting unit can also consist of or comprises a droplet ejecting unit driven by supersonic or piezo driven. The pipetting unit can pipett samples as well as supporting liquids.

In embodiments of the first aspect of the present invention, the pipetting unit is arranged after the sample delivery unit and before the sample analytical unit. The sample analytical unit can also consist of or comprises several independend reagent pipetting units which are independend of the mentioned pipetting unit.

In embodiments of the first aspect of the invention, the fluid transport unit is for adding/removing fluids, such as samples, reagents, wash fluids, suspension fluids, into/from the reaction container(s) by delivering the reagents through tubings.

In embodiments of the first aspect of the present invention, the fluid transport unit is arranged after the sample delivery unit and before the sample analytical unit.

In embodiments of the first aspect of the present invention, the one or more pipetting units and/or fluid transport units are for adding, mixing or removing fluids.

In embodiments of the first aspect of the invention, the reaction container transporting mechanism unit. The transport can be achieved by convoyer or a gripping system which can handle reaction vessels.

In embodiments of the first aspect of the present invention, the reaction container transporting mechanism unit is for delivering sample or to the following process station, preferably the ion generation station.

In embodiments of the first aspect of the present invention, the reaction container transporting mechanism unit is arranged after the sample delivery unit and before the sample analytical unit.

In embodiments of the first aspect of the invention, the sample pre-treatment unit comprises the enrichment handling unit. The enrichment can be performed by concentration of the sample through reducing the volume of solvent by e.g. evaporation or by attaching the analyte on a solid supported microparticles. Another enrichment possibility is a precipitation of the analyte in its surrounding liquid by e.g. changing temperature or solvent exchange by following solid-liquid separation. In embodiments of the first aspect of the invention, the sample pre-treatment unit comprises the derivatization unit which consists of or comprises a pipetting unit with heating and cooling capabilities. In this unit, a derivatization reagent or derivatization reagents can be used.

In embodiments of the first aspect of the invention, the derivatization reagents is selected from the group consisting of: dansylchloride, carbamic acid, N-[2-[[[2-(diethylamino)ethyl]amino]carbonyl]-6-quinolinyl]-, 2,5-dioxo-1-pyrrolidinyl ester (RapiFluor-MS), 4-substituted 1,2,4-triazoline-3,5-diones (Cookson-type reagents), 4-Phenyl-1,2,4-triazolin-3,5-dion-derivative (Amplifex Diene), 1-propanaminium, 3-(aminooxy)-N,N,N-trimethyl-compound comprising an appropriate counter ion, e.g. bromide, chloride, iodine, etc. (Amplifex Keto), acethydrazide trimethylammonium chloride (Girard T), 1-(carboxymethyl)pyridinium chloride hydrazide (Girard P) and pyridiyl amine.

In embodiments of the first aspect of the present invention, the enrichment handling unit is arranged after the sample delivery unit and before the sample analytical unit.

In embodiments of the first aspect of the invention, the sample pre-treatment unit comprises the solvent evaporation unit. The solvent evaporation unit can be part of the enrichment handling unit.

In embodiments of the first aspect of the present invention, the solvent evaporation unit is arranged after the sample delivery unit and before the sample analytical unit.

In embodiments of the first aspect of the present invention, the addition of an hemolysis reagent can be performed in the sample pre-treatment unit.

In embodiments of the first aspect of the present invention, the addition of enzymatic reagents to the sample, e.g. urine sample, can be performed in the sample pre-treatment unit.

In embodiments of the first aspect of the present invention, the addition of at least one derivatisation reagent to the sample, e.g. urine sample, can be performed in the sample pre-treatment unit.

In embodiments of the first aspect of the invention, the analyte of interest comprises a functional group. The functional group is capable of reacting with a reactive unit Q of the derivatisation reagent.

In embodiments of the first aspect of the present invention, the functional group is selected from the group consisting of carbonyl group, diene group, hydroxyl group, amine group, imine group, ketone group, aldehyde group, thiol group, diol group, phenolic group, expoxid group, disulfide group, nucleobase group, carboxylic acid group, terminal cysteine group, terminal serine group and azide group.

In embodiments of the first aspect of the present invention, the analyte molecule comprises a carbonyl group as functional group which is selected from the group consisting of a carboxylic acid group, aldehyde group, keto group, a masked aldehyde, masked keto group, ester group, amide group, and anhydride group. Aldoses (aldehyde and keto) exist as acetal and hemiacetals, a sort of masked form of the parent aldehyde/ keto.

In embodiments of the first aspect of the present invention, the carbonyl group is an amide group, the skilled person is well aware that the amide group as such is a stable group, but that it can be hydrolyzed to convert the amide group into an carboxylic acid group and an amino group. Hydrolysis of the amide group may be achieved via acid/base catalysed reaction or by enzymatic process either of which is well-known to the skilled person. In embodiments of the first aspect of the present invention, wherein the carbonyl group is a masked aldehyde group or a masked keto group, the respective group is either a hemiacetal group or acetal group, in particular a cyclic hemiacetal group or acetal group. In embodiments of the first aspect of the present invention, the acetal group, is converted into an aldehyde or keto group before reaction with the compound.

In embodiments of the first aspect of the present invention, the carbonyl group is a keto group. In embodiments of the first aspect of the present invention, the keto group may be transferred into an intermediate imine group before reacting with the reactive unit of compounds. In embodiments of the first aspect of the present invention, the analyte molecule comprising one or more keto groups is a ketosteroid. In particular embodiments of the first aspect of the present invention, the ketosteroid is selected from the group consisting of testosterone, epitestosterone, dihydrotestosterone (DHT), desoxymethyltestosterone (DMT), tetrahydrogestrinone (THG), aldosterone, estrone, 4-hydroxyestrone, 2-methoxyestrone, 2-hydroxyestrone, 16-ketoestradiol, 16-alpha-hydroxyestrone, 2-hydroxyestrone-3-methylether, prednisone, prednisolone, pregnenolone, progesterone, dehydroepiandrosterone (DHEA), 17-hydroxypregnenolone, 17-hydroxyprogesterone, androsterone, epiandrosterone, Δ4-androstenedione, 11-deoxycortisol, corticosterone, 21-deoxycortisol, 11-deoxycorticosterone, allopregnanolone and aldosterone.

In embodiments of the first aspect of the present invention, the carbonyl group is a carboxyl group. In embodiments of the first aspect of the present invention, the carboxyl group reacts directly with the compound or it is converted into an activated ester group before reaction with the compound. In embodiments of the first aspect of the present invention, the analyte molecule comprising one or more carboxyl groups is selected from the group consisting of Δ8-tetrahydrocannabinolic acid, benzoylecgonin, salicylic acid, 2-hydroxybenzoic acid, gabapentin, pregabalin, valproic acid, vancomycin, methotrexate, mycophenolic acid, montelukast, repaglinide, furosemide, telmisartan, gemfibrozil, diclofenac, ibuprofen, indomethacin, zomepirac, isoxepac and penicillin. In embodiments of the first aspect of the present invention, the analyte molecule comprising one or more carboxyl groups is an amino acid selected from the group consisting of arginine, lysine, aspartic acid, glutamic acid, glutamine, asparagine, histidine, serine, threonine, tyrosine, cysteine, tryptophan, alanine, isoleucine, leucine, methionine, phenyalanine, valine, proline and glycine.

In embodiments of the first aspect of the present invention, the carbonyl group is an aldehyde group. In embodiments of the first aspect of the present invention, the aldehyde group may be transferred into an intermediate imine group before reacting with the reactive unit of compounds. In embodiments of the first aspect of the present invention, the analyte molecule comprising one or more aldehyde groups is selected from the group consisting of pyridoxal, N-acetyl-D-glucosamine, alcaftadine, streptomycin and josamycin.

In embodiments of the first aspect of the present invention, the carbonyl group is an carbonyl ester group. In embodiments of the first aspect of the present invention, the analyte molecule comprising one or more ester groups is selected from the group consisting of cocaine, heroin, Ritalin, aceclofenac, acetylcholine, amcinonide, amiloxate, amylocaine, anileridine, aranidipine artesunate and pethidine.

In embodiments of the first aspect of the present invention, the carbonyl group is an anhydride group. In embodiments of the first aspect of the present invention, the analyte molecule comprising one or more anhydride groups is selected from the group consisting of cantharidin, succinic anhydride, trimellitic anhydride and maleic anhydride.

In embodiments of the first aspect of the present invention, the analyte molecule comprises one or more diene groups, in particular to conjugated diene groups, as functional group. In embodiments of the first aspect of the present invention, the analyte molecule comprising one or more diene groups is a secosteroid. In embodiments, the secosteroid is selected from the group consisting of cholecalciferol (vitamin D3), ergocalciferol (vitamin D2), calcifediol, calcitriol, tachysterol, lumisterol and tacalcitol. In particular, the secosteroid is vitamin D, in particular vitamin D2 or D3 or derivates thereof. In particular embodiments, the secosteroid is selected from the group consisting of vitamin D2, vitamin D3, 25-hydroxyvitamin D2, 25-hydroxyvitamin D3 (calcifediol), 3-epi-25-hydroxyvitamin D2, 3-epi-25-hydroxyvitamin D3, 1,25-dihydroxyvitamin D2, 1,25-dihydroxyvitamin D3 (calcitriol), 24,25-dihydroxyvitamin D2, 24,25-dihydroxyvitamin D3. In embodiments of the first aspect of the present invention, the analyte molecule comprising one or more diene groups is selected from the group consisting of vitamin A, tretinoin, isotretinoin, alitretinoin, natamycin, sirolimus, amphotericin B, nystatin, everolimus, temsirolimus and fidaxomicin.

In embodiments of the first aspect of the present invention, the analyte molecule comprises one or more hydroxyl group as functional group. In embodiments of the first aspect of the present invention, the analyte molecule comprises a single hydroxyl group or two hydroxyl groups. In embodiments wherein more than one hydroxyl group is present, the two hydroxyl groups may be positioned adjacent to each other (1,2-diol) or may be separated by 1, 2 or 3 C atoms (1,3-diol, 1,4-diol, 1,5-diol, respectively). In particular embodiments of the first aspect, the analyte molecule comprises a 1,2-diol group. In embodiments, wherein only one hydroxyl group is present, said analyte is selected from the group consisting of primary alcohol, secondary alcohol and tertiary alcohol. In embodiments of the first aspect of the present invention, wherein the analyte molecule comprises one or more hydroxyl groups, the analyte is selected from the group consisting of benzyl alcohol, menthol, L-carnitine, pyridoxine, metronidazole, isosorbide mononitrate, guaifenesin, clavulanic acid, Miglitol, zalcitabine, isoprenaline, aciclovir, methocarbamol, tramadol, venlafaxine, atropine, clofedanol, alpha-hydroxyalprazolam, alpha-Hydroxytriazolam, lorazepam, oxazepam, Temazepam, ethyl glucuronide, ethylmorphine, morphine, morphine-3-glucuronide, buprenorphine, codeine, dihydrocodeine, p-hydroxypropoxyphene, O-desmethyltramadol, Desmetramadol, dihydroquinidine and quinidine. In embodiments of the first aspect of the present invention, wherein the analyte molecule comprises more than one hydroxyl groups, the analyte is selected from the group consisting of vitamin C, glucosamine, mannitol, tetrahydrobiopterin, cytarabine, azacitidine, ribavirin, floxuridine, Gemcitabine, Streptozotocin, adenosine, Vidarabine, cladribine, estriol, trifluridine, clofarabine, nadolol, zanamivir, lactulose, adenosine monophosphate, idoxuridine, regadenoson, lincomycin, clindamycin, Canagliflozin, tobramycin, netilmicin, kanamycin, ticagrelor, epirubicin, doxorubicin, arbekacin, streptomycin, ouabain, amikacin, neomycin, framycetin, paromomycin, erythromycin, clarithromycin, azithromycin, vindesine, digitoxin, digoxin, metrizamide, acetyldigitoxin, deslanoside, Fludarabine, clofarabine, gemcitabine, cytarabine, capecitabine, vidarabine, and plicamycin.

In embodiments of the first aspect of the present invention, the analyte molecule comprises one or more thiol group (including but not limited to alkyl thiol and aryl thiol groups) as functional group. In embodiments of the first aspect of the present invention, the analyte molecule comprising one or more thiol groups is selected from the group consisting of thiomandelic acid, DL-captopril, DL-thiorphan, N-acetylcysteine, D-penicillamine, glutathione, L-cysteine, zofenoprilat, tiopronin, dimercaprol, succimer.

In embodiments of the first aspect of the present invention, the analyte molecule comprises one or more disulfide group as functional group. In embodiments of the first aspect of the present invention, the analyte molecule comprising one or more disulfide groups is selected from the group consisting of glutathione disulfide, dipyrithione, selenium sulfide, disulfiram, lipoic acid, L-cystine, fursultiamine, octreotide, desmopressin, vapreotide, terlipressin, linaclotide and peginesatide. Selenium sulfide can be selenium disulfide, SeS₂, or selenium hexasulfide, Se₂S₆.

In embodiments of the first aspect of the present invention, the analyte molecule comprises one or more epoxide group as functional group. In embodiments of the first aspect of the present invention, the analyte molecule comprising one or more epoxide groups is selected from the group consisting of Carbamazepine-10,11-epoxide, carfilzomib, furosemide epoxide, fosfomycin, sevelamer hydrochloride, cerulenin, scopolamine, tiotropium, tiotropium bromide, methylscopolamine bromide, eplerenone, mupirocin, natamycin, and troleandomycin.

In embodiments of the first aspect of the present invention, the analyte molecule comprises one or more phenol groups as functional group. In particular embodiments of the first aspect of the present invention, analyte molecules comprising one or more phenol groups are steroids or steroid-like compounds. In embodiments of the first aspect of the present invention, the analyte molecule comprising one or more phenol groups is a steroid or a steroid-like compound having an A-ring which is sp² hybridized and an OH group at the 3 -position of the A-ring. In particular embodiments of the first aspect of the present invention, the steroid or steroid-like analyte molecule is selected from the group consisting of estrogen, estrogen-like compounds, estrone (El), estradiol (E2), 17a-estradiol, 17b-estradiol, estriol (E3), 16-epiestriol, 17-epiestriol, and 16, 17-epiestriol and/or metabolites thereof. In embodiments, the metabolites are selected from the group consisiting of estriol, 16-epiestriol (16-epiE3), 17-epiestriol (17-epiE3), 16,17-epiestriol (16,17-epiE3), 16-ketoestradiol (16-ketoE2), 16a-hydroxyestrone (16a-OHEl), 2-methoxyestrone (2-MeOE1), 4-methoxyestrone (4-MeOE1), 2-hydroxyestrone-3-methyl ether (3-MeOE1), 2-methoxyestradiol (2-MeOE2), 4-methoxyestradiol (4-MeOE2), 2-hydroxyestrone (2-OHE1), 4-hydroxyestrone (4-OHE1), 2-hydroxyestradiol (2-OHE2), estrone (El), estrone sulfate (Els), 17a- estradiol (E2a), 17b-estradiol (E2B), estradiol sulfate (E2S), equilin (EQ), 17a-dihydroequilin (EQa), 17b-dihydroequilin (EQb), Equilenin (EN), 17-dihydroequilenin (ENa), 17α-dihydroequilenin, 17β-dihydroequilenin (ENb) , Δ8,9-dehydroestrone (dEl), Δ8,9-dehydroestrone sulfate (dEls), Δ9-tetrahydrocannabinol, mycophenolic acid. β or b can be used interchangeable. α and a can be used interchangeable.

In embodiments of the first aspect of the present invention, the analyte molecule comprises an amine group as functional group. In embodiments of the first aspect of the present invention, the amine group is an alkyl amine or an aryl amine group. In embodiments of the first aspect of the present invention, the analyte comprising one or more amine groups is selected from the group consisting of proteins and peptides. In embodiments of the first aspect of the present invention, the analyte molecule comprising an amine group is selected from the group consisting of 3,4-methylenedioxyamphetamine, 3,4-methylenedioxy-N-ethylamphetamine, 3,4-methylenedioxymethamphetamine, Amphetamine, Methamphetamine, N-methyl-1,3-benzodioxolylbutanamine, 7-aminoclonazepam, 7-aminoflunitrazepam, 3,4-dimethylmethcathinone, 3-fluoromethcathinone, 4-methoxymetheathinone, 4-methyletheathinone, 4-methylmethcathinone, amfepramone, butylone, etheathinone, elephedrone, methcathinone, methylone, methylenedioxypyrovalerone, benzoylecgonine, dehydronorketamine, ketamine, norketamine, methadone, normethadone, 6-acetylmorphine, diacetylmorphine, morphine, norhydrocodone, oxycodone, oxymorphone, phencyclidine, norpropoxyphene, amitriptyline, clomipramine, dothiepin, doxepin, imipramine, nortriptyline, trimipramine, fentanyl, glycylxylidide, lidocaine, monoethylglycylxylidide, N-acetylprocainamide, procainamide, pregabalin, 2-Methylamino-1-(3,4-methylendioxyphenyl)butan, N-methyl-1,3-benzodioxolylbutanamine, 2-Amino-1-(3,4-methylendioxyphenyl)butan, 1,3-benzodioxolylbutanamine, normeperidine, O-Destramadol, desmetramadol, tramadol, lamotrigine, Theophylline, amikacin, gentamicin, tobramycin, vancomycin, Methotrexate, Gabapentin sisomicin and 5-methylcytosine.

In embodiments of the first aspect of the present invention, the analyte molecule is a carbohydrate or substance having a carbohydrate moiety, e.g. a glycoprotein or a nucleoside. In embodiments of the first aspect of the present invention, the analyte molecule is a monosaccharide, in particular selected from the group consisting of ribose, desoxyribose, arabinose, ribulose, glucose, mannose, galactose, fucose, fructose, N-acetylglucosamine, N-acetylgalactosamine, neuraminic acid, N-acetylneurominic acid, etc.. In embodiments, the analyte molecule is an oligosaccharide, in particular selected from the group consisting of a disaccharide, trisaccharid, tetrasaccharide, polysaccharide. In embodiments of the first aspect of the present invention, the disaccharide is selected from the group consisting of sucrose, maltose and lactose. In embodiments of the first aspect of the present invention, the analyte molecule is a substance comprising above described mono-, di-, tri-, tetra-, oligo- or polysaccharide moiety.

In embodiments of the first aspect of the present invention, the analyte molecule comprises an azide group as functional group which is selected from the group consisting of alkyl or aryl azide. In embodiments of the first aspect of the present invention, the analyte molecule comprising one or more azide groups is selected from the group consisting of zidovudine and azidocillin.

Such analyte molecules may be present in biological or clinical samples such as body liquids, e.g. blood, serum, plasma, urine, saliva, spinal fluid, etc., tissue or cell extracts, etc. In embodiments of the first aspect of the present invention, the analyte molecule(s) are present in a biological or clinical sample selected from the group consisting of blood, serum, plasma, urine, saliva, spinal fluid, and a dried blood spot. In some embodiments of the first aspect of the present invention, the analyte molecules may be present in a sample which is a purified or partially purified sample, e.g. a purified or partially purified protein mixture or extract.

In embodiments of the first aspect of the present invention, the analyte of interest is a steroid.

In embodiments of the first aspect of the present invention, the steroid is selected from the group consisting of Cortisol, DHEA-S, Estradiol, Progesterone, Testosterone, 17-Hydroxyprogesterone, Aldosterone, Androstendione, DHEA, Dihydrotestosterone and Cortisone.

In embodiments of the first aspect of the present invention, the analyte of interest is a Vitamin D.

In embodiments of the first aspect of the present invention, the Vitamin D is selected from the group consisting of 25(OH)D2, 25(OH)D3, 24,25(OH)2D2, 24,25(OH)2D3, 1,25(OH)2D2 and 1,25(OH)2D3. A skilled person knows the abbreviations mentioned above for Vitamin D.

In embodiments of the first aspect of the present invention, the analyte of interest is selected from the group consisting of Cyclosporine A, Everolimus, Sirolimus, Tacrolimus, Acetaminophen, Salicylate, Theophylline and Digoxin.

In embodiments of the first aspect of the present invention, the analyte of interest is selected from the group consisting of Phenytoin, Valproic acid, Phenytoin, Levetiracetam, Carbamazepine, Carbamazepine-10,11-epoxide, Phenobarbital, Primidone, Gabapentin, Zonisamid, Lamotrigine and Topiramateand.

In embodiments of the first aspect of the present invention, the analyte of interest is selected from the group consisting of Gentamicin, Tobramycin, Amikacin, Vancomycin, Piperacilline (Tazobactam), Meropenem and Linezolid.

In embodiments of the first aspect of the present invention, the analyte of interest is selected from the group consisting of Methotrexate, Voriconazole, Mycophenolic acid and Mycophenolic acid-glucuronide.

In embodiments of the first aspect of the present invention, the analyte of interest is selected from the group consisting of Buprenorphine, 6-monoacatylmorphine, Codeine, Dihydrocodeine, Morphine, Morphine-3-glucuronide and Tramadol.

In embodiments of the first aspect of the present invention, the analyte of interest is selected from the group consisting of Acetylfentanyl, Carfentanil, Fentanyl, Hydrocodone, Norfentanyl, Oxycodone and Oxymorphone.

In embodiments of the first aspect of the present invention, the sample preparation station comprises a sample analytical unit.

In embodiments of the first aspect of the present invention, the sample analytical unit comprises at least one unit or more than one units or a combination of the units selected from the group: a solid-liquid support analyte enrichment unit, solid-liquid support matrix depletion unit, gaseous sample enrichment or matrix separation unit, solid sample on solid support unit.

In embodiments of the first aspect of the present invention, the solid-liquid support analyte enrichment unit is e.g. SPE (solid phase extraction), SLE (Solid liquid extraction), SPME (Solid Phase Micro Extraction). This unit brings the sample in contact to a supporting liquid or uses the liquid in which the sample is already in. Furthermore a solid gets into contact with the sample and due to surface adsorption and or absorption a separation of compunds from the sample is achieved. The solid can be removed and the supernatant or the solid particles can be processed further by e.g. washing and/or extraction. The unit is capable to handle this type of extraction in an automated way from sample in, extraction and or washing followed by transferring the processed sample further.

In embodiments of the first aspect of the present invention, the the solid-liquid support analyte enrichment unit is selected form the group consisting of solid-phase extraction (SPE), micro particle, liquid-liquid extraction (LLE), liquid chromatography (LC), high pressure liquid chromatography (HPLC) and combinations thereof.

In embodiments of the first aspect of the present invention, the solid-liquid support matrix depletion unit. This unit is capable in a similar manner to the solid-liquid support analyte enrichment unit to remove unwanted matrix components from the sample by e.g. microparticles or a prepacked column. The unwanted matrix is by this unit bound to the particles or gets washed within the supernatenat of the particles.

In embodiments of the first aspect of the present invention, the solid-liquid support matrix depletion unit is selected form the group consisting of solid-phase extraction (SPE), micro particle and liquid-liquid extraction (LLE).

In embodiments of the first aspect of the present invention, the gaseous sample enrichment or matrix separation unit consist of or comprises a SLE unit or a heating/cooling unit in which analytes and/or matrix compounds within a analytical sample gets separated. Further, more filtering devices or units to separate aerosols e.g. water droplets ranging from 10 nm-10 mm get filtered off.

In embodiments of the first aspect of the present invention, the solid sample on solid support unit consists of or comprises a pipetting unit if liquid samples are handled or a robotic arm for solid samples. The solid support can consist of or comprises a metal plate or a solid strip. The sample can be dried if a liquid was placed and/or mixed on the solid support with other reagents. An example is the MALDI (matrix assisted laser desorption) in which a pre mixed analyte and matrix compounds e.g. DHB are pipetted to get onto a steel plate and evaporate till dryness follows by laser irridation and mass spectrometrical analysis.

In embodiments of the first aspect of the present invention, the solid sample on solid support unit is imaging of cross sections and mineral analysis.

In embodiments of the first aspect of the present invention, the gaseous sample enrichment or matrix separation unit is inert gas stripping or headspace extraction or gas adsorption supported by liquid and/or solid e.g. activated charcoal or combinations thereof.

The clinical diagnostic system comprises a ion generation station. The ion generation station is for generating at least one ion or ions of the at least one analyte of interest.

In embodiments of the first aspect of the present invention, the ion or ions of the at least one analyte of interest are in the gaseous state, preferably after leaving the ion generation station.

In embodiments of the first aspect of the present invention, the ion generation station is arranged between the sample preparation station and the at least one selectivity enhancer station. This can mean that the ion generation station is coupled with the sample preparation station and is coupled to the at least one selectivity enhancer station.

In embodiments of the first aspect of the present invention, the ion generation station comprises an unsteady analyte ion supply unit and/or a steady analyte ion supply unit.

In embodiments of the first aspect of the present invention, the the unsteady analyte ion supply unit is selected from the group consisting of laser desorption ionzation (LDI), dielectric barrier liquid flow, surface plasma ionization and combinations thereof.

In embodiments of the first aspect of the present invention, the laser desorption ionzation (LDI) is a system which generates ion/ ionization process driven by the use of the laser energy. The energy is taken up by the molecule and/or a supporting material to separate charges and gererate ions.

In embodiments of the first aspect of the present invention, the dielectric barrier liquid flow are characterized by the presence of one or more insulating layers (dielectric barriers) in the current path between metal electrodes in addition to the discharge space. The dielectric barriers usually use several common insulation materials, for example glass or silica glass, ceramic materials, mica, and thin enamel or polymer layers. There are two basic configurations for a DBD: planar and annular.

In embodiments of the first aspect of the present invention, the surface plasma ionization is made of or comprises a source which provides a cold plasma by e.g. electrical discharge which is pointed towards a surface bound analyte. The analyte gets ionized by the cold plasma inherent ions.

In embodiments of the first aspect of the present invention, the the steady analyte ion supply unit is selected from the group consisting of flow injection (preferably flow injection using ESI or APCI or Nano Spray), paper spray, electron ionization (EI), matrix assisted ionization (MAI), chemical ionization (CI), e.g. proton-transfer-reaction (PTR), radioactive supported ionization, e.g. using ⁶³Ni and combinations thereof.

In embodiments of the first aspect of the present invention, the flow injection relates to a direct sample to ESI/ APCI spray needle contact. The sample gets without any further processing through the ion source.

In embodiments of the first aspect of the present invention, the flow injection is an electrospray ionization (ESI).

In embodiments of the first aspect of the present invention, the ESI is nanoESI.

In embodiments of the first aspect of the present invention, the nanoESI is static nanoESI.

In embodiments of the first aspect of the present invention, the flow injection is atmospheric pressure chemical ionization (APCI).

In embodiments of the first aspect of the present invention, the the clinical diagnostic system comprises high-field asymmetric-waveform ion-mobility spectrometry (FAIMS).

In embodiments of the first aspect of the present invention, the paper spray relates to a paper strip which is shaped on one side with a tip or cone. The analyte is applied on the tip surface and wetted with solvent. On the opposite side of the paper a high voltage is applied (around 0,5-5kV) and the tip is place in front of the mass spectrometer.

In embodiments of the first aspect of the present invention, the electron ionization (EI) uses free electrons from a glowing wire which are accelerated by an electromagnetic field. The electron beam hits the target analyte and ionizes it therefore by forming radical cations and or anions.

In embodiments of the first aspect of the present invention, the matrix assisted ionization (MAI) uses a solide matrix e.g. 3-NBN which dissociates by thermal energy and or electric fields and therefore ionized the molecule of interest which is in close contact to the matrix (e.g. by co-precipitation).

In embodiments of the first aspect of the present invention, the chemical ionization (CI) uses an electron beam to ionize a supporting gas (e.g. methane or ammonia) which further ionizes the target analyte.

In embodiments of the first aspect of the present invention, the radioactive supported ionization uses the alpha radiation of e.g. ⁶³Ni as source for ions which therefore ionizes a target molecule within the radion beam of the radioactive material.

The clinical diagnostic system comprises a at least one selectivity enhancer station. The clinical diagnostic system can comprise more than one selectivity enhancer station, e.g. 2 or 3 or 4 or 5 or 6, for enhancing the selectivity of the at least one analyte of interest. In case of more than 2 selectivity enhancer station, the selectivity enhancer stations are coupled to each other one after the other. The at least one selectivity enhancer station separates ions based on the respective ion size. The at least one selectivity enhancer station comprises an ion mobility unit for separation ions with respect to their ion mobility using electric and/or radio frequency fields (AC/DC and/or RF) applied to one or more electrodes.In embodiments of the first aspect of the present invention, the clinical diagnostic system comprises exactly one selectivity enhancer station.

In embodiments of the first aspect of the present invention, the at least one selectivity enhancer station comprises at least one ion storage unit and/or the at least one ion transportation unit, and an ion selection unit which separates ions based on their m/z (mass over charge ratio). Preferably, the ion storage unit and/or the at least one ion transportation unit has no ion separation capability.

In embodiments of the first aspect of the present invention, the at least one selectivity enhancer station comprises exactly one ion storage unit and/or exactly one ion transportation unit.

In embodiments of the first aspect of the present invention, the ions with similar or equal ion mobility unit bunch together in the ion mobility unit.

In embodiments of the first aspect of the present invention, the ion mobility unit performs a gas phase separation of a mixture of ions.

In embodiments of the first aspect of the present invention, the ions have a mass to charge ratio the range of 1 to about 100,000 in the ion mobility unit.

In embodiments of the first aspect of the present invention, the ions have a drift time through the ion mobility unit of about 0 to about 60 seconds.

In embodiments of the first aspect of the present invention, the at least a portion of the ion mobility unit is maintained at a pressure in the range of about 10-3 torr to atmospheric pressure.

In embodiments of the first aspect of the present invention, the ions in the ion mobility unit are formed by using at least one of the following: photoionization, Corona discharge, laser ionization, electron impact, field ionization, chemical ionization, and electrospray.

In embodiments of the first aspect of the present invention, the ions are formed outside of the ion mobility unit, preferably in the ion generation station.

In embodiments of the first aspect of the present invention, the ion mobility unit comprises a non-constant electric field applied to the ion mobility unit to form a potential gradient along a portion of the ion mobility unit so that ions with similar mobilities bunch together into sharper peaks while maintaining separation between other ions.

In embodiments of the first aspect of the present invention, the potential gradient is a DC gradient.

In embodiments of the first aspect of the present invention, the potential gradient progressively increases or decreases along the length of the device.

In embodiments of the first aspect of the present invention, the potential gradient along a length of the device is between 0 to about 5,000 volts/mm.

In embodiments of the first aspect of the present invention, the ion mobility unit uses an electric field and/or a radio frequency fields (AC/DC and/or RF) applied to one or more electrodes.

In embodiments of the first aspect of the present invention, the ion mobility unit performs the Structures for Lossless Ion Manipulation technology (SLIM). SLIM technology separates ions and applies electric fields to electrodes on conventional parallel printed circuit board surfaces creating ion conduits that can separate and move ions losslessly. Thus, the ions in the sample can be separated with a high resolution and high troughput. The SLIM technique is known for a skilled person, e.g. from WO 2017/062102 A1 (EP3359960) or WO2014/168660 Al (EP2984675), wherein the arrangements and functions of this technique is incorporated herein by reference.

In embodiments of the first aspect of the present invention, the ion mobility unit comprises a
- first pair of opposing electrode arrangements coupled to a voltage source and that confines ions between the first pair opposing electrode arrangements in a confinement volume portion of a confinement volume inwardly laterally in a first confinement direction with respect to a longitudinal ion propagation direction, each opposing electrode arrangement of the first pair including an arrangement of RF electrodes that receives an unbiased RF voltage from the voltage source having an alternate phase between adjacent RF electrodes of the arrangement of RF electrodes of the opposing electrode arrangement of the first pair that provides the confining of ions between the first pair of opposing electrode arrangements; and
- a second pair of opposing electrode arrangements coupled to the voltage source and separate from the first pair of opposing electrode arrangements and that confines the ions between the second pair of opposing electrode arrangements in the confinement volume inwardly laterally in a second confnement direction that complements the first confinement direction, each opposing electrode arrangement of the second pair including an arrangement of RF electrodes that receives an unbiased RF voltage from the voltage source having an alternate phase between adjacent RF electrodes of the arrangement of RF electrodes of the opposing electrode arrangement of the second pair; wherein the confnement volume defines a first ion conduit and the apparatus further comprises a second ion conduit separate and laterally spaced apart from the first ion conduit and wherein the second ion conduit includes a plurality of electrode arrangements and at least one the electrode arrangements of the second ion conduit is an opposing electrode arrangement of the first or second pairs of opposing electrode arrangements of the first ion conduit.

In embodiments of the first aspect of the present invention, the ion mobility unit comprises:
at least one surface;
a first plurality of continuous electrodes coupled to the at least one surface and in electrical communication with a radiofrequency (RF) voltage source, wherein an RF voltage applied to adjacent electrodes of the first plurality of electrodes by the RF voltage source is phase shifted on the adjacent electrodes of the first plurality of electrodes by approximately 180°; and
a second plurality of segmented electrodes coupled to the at least one surface and arranged in longitudinal sets between or adjacent to the first plurality of electrodes, the second plurality of segmented electrodes being further in electrical communication with an alternating current (AC) voltage source, wherein an AC voltage waveform applied to adjacent electrodes within a longitudinal set of the second plurality of segmented electrodes by the AC voltage source is phase shifted on the adjacent electrodes of the second plurality of electrodes by 1°-359°.

In embodiments of the first aspect of the present invention, the ion mobility unit comprises a plurality of guard electrodes positioned on outer ends of the first and second plurality of electrodes on the at least one surface, the plurality of guard electrodes being further in electrical communication with a DC voltage source, wherein the plurality of guard electrodes generate electric fields that constrain ion motion towards the guard electrodes when receiving a constant DC voltage from the DC voltage source.

In embodiments of the first aspect of the present invention, the AC voltage waveform is a sine wave.

In embodiments of the first aspect of the present invention, the AC voltage waveform is the sum of more than one AC voltage waveform.

In embodiments of the first aspect of the present invention, the AC voltage waveform applied to adjacent electrodes within a longitudinal set of the second plurality of segmented electrodes is phase shifted on the adjacent electrodes of the second plurality of segmented electrodes in a repeating pattern.

In embodiments of the first aspect of the present invention, the AC voltage waveform applied to adjacent electrodes within a longitudinal set of the second plurality of segmented electrodes is phase shifted by approximately 45°, 90° or 120° on the adjacent electrodes of the second plurality of electrodes in a stepwise fashion.

In embodiments of the first aspect of the present invention, the at least one surface comprises a single and non-planar surface.

In embodiments of the first aspect of the present invention, the single, non-planar surface is one of the following shapes: curved, cylindrical, a spiral, a funnel, hemispherical, or elliptical.

In embodiments of the first aspect of the present invention, the at least one surface comprises two surfaces spaced apart from one another.

In embodiments of the first aspect of the present invention, the the two surfaces are approximately parallel to one another.

In embodiments of the first aspect of the present invention, a frequency of the applied AC voltage waveform is selected from the range of 10 Hz - 200 kHz, and a frequency of the applied RF voltage is selected from the range of 100 kHz - 5 MHz.

In embodiments of the first aspect of the present invention, a frequency applied AC voltage waveform is selected from the range of 1 Hz to 1 kHz.

In embodiments of the first aspect of the present invention, a pressure range of the ion mobility unit is from atmospheric pressure to 1 mtorr vacuum.

In embodiments of the first aspect of the present invention, the ion mobility unit comprises
at least one surface;
a plurality of segmented electrodes coupled to the at least one surface and arranged in one or more longitudinal sets, the plurality of segmented electrodes being in electrical communication with an alternating current (AC) voltage source and a radiofrequency (RF) voltage source;
wherein an AC voltage waveform applied to adjacent electrodes within a longitudinal set of the plurality of electrodes by the AC voltage source is phase shifted by 1°-359°; and
wherein an RF voltage applied to adjacent electrodes of the plurality of electrodes by the RF voltage source is phase shifted by approximately 180°.

In embodiments of the first aspect of the present invention.

In embodiments of the first aspect of the present invention, the ion mobility unit comprises a supporting gas, preferably with variable direction to the AC/DC and RF driven ion direction can be applied with angle towards the ion flow of 1° to 359°. Preferably, the angle towards the ion flow is 90° or 180°.

In embodiments of the first aspect of the present invention, the supporting gas is inert.

In embodiments of the first aspect of the present invention, the supporting gas is a non reactive gas, e.g. nitrogen, argon, xenon, krypton, SF6 and/or helium.

In embodiments of the first aspect of the present invention, the supporting gas is non reactive with pressure of 0-1 bar, preferably, 1 mbar - 500 mbar. By flowing the supporting gas the ions can be further separated with this alternative force toward the AC/DC RF driven ion movement.

In embodiments of the first aspect of the present invention, the ion mobility unit comprises
a. pair of surfaces;
b. arrays of electrodes coupled to the surfaces to which RF potentials are applied to at least one of the surfaces in order to create a pseudopotential that inhibits charged particles from approaching either of the surfaces; and
c. simultaneous application of DC potentials to control and restrict movement of ions in between the surfaces.
the arrays of electrodes comprise an array of inner electrodes, a first outer electrode array, and a second outer electrode array, wherein the inner and the outer array of electrodes extend substantially along the length of each surface.

In embodiments of the first aspect of the present invention, the arrays of electrodes comprise an array of inner electrodes, a first outer electrode array, and a second outer electrode array, wherein the inner and the outer array of electrodes extend substantially along the length of each surface.

In embodiments of the first aspect of the present invention, the first outer electrode array is positioned on one side of the array of inner electrodes, and the second outer electrode array is positioned on the other side of the array of inner electrodes.

In embodiments of the first aspect of the present invention, the DC potentials are applied to the first and second outer electrode arrays and wherein the RF potentials, with a superimposed electric field, are applied to the array of inner electrodes.

In embodiments of the first aspect of the present invention, the RF potentials are applied along with the DC potentials on the first and second outer electrode arrays.

In embodiments of the first aspect of the present invention, the RF potentials are applied to only one of the two surfaces.

In embodiments of the first aspect of the present invention, the RF potentials are applied to both of the two surfaces.

In embodiments of the first aspect of the present invention, the RF on at least one inner electrode is out of phase with its neighboring electrode.

In embodiments of the first aspect of the present invention, each inner electrode is phase shifted with its neighboring inner electrode to form the pseudopotential.

In embodiments of the first aspect of the present invention, the inner electrode is approximately 180 degrees out of phase with its neighboring inner electrode to form the pseudopotential.

In embodiments of the first aspect of the present invention, the ion mobility unit further comprises multiple pairs of surfaces, wherein transfer of the ions is allowed through an aperture and guided by a series of electrodes to move between different pairs of surfaces of the multiple pairs of surfaces.

In embodiments of the first aspect of the present invention, the series of electrodes have RF potential of alternating polarity to prevent loss of ions as the ions move between the different pairs of parallel surfaces.

In embodiments of the first aspect of the present invention, the electrodes on the pair of surfaces form at least one of the following configurations:
a. a substantially T-shaped configuration, allowing ions to be switched at a junction of the T-shaped configuration;
b. a substantially Y-shaped configuration, allowing ions to be switched at a junction of the Y-shaped configuration;
c. a substantially X-shaped or cross-shaped configuration, allowing ions to be switched at a junction of one or more sides of the X-shaped or cross-shaped configuration; and d. a substantially multidirectional shape, such as an asterisk (*) -shaped configuration, with multiple junction points, allowing ions to be switched at a junction to one or more sides of the configuration.

In embodiments of the first aspect of the present invention, the electric field allows the ions to move in a circular-shaped path, a rectangular-shaped path, or other irregular path, to allow the ions to make more than one transit.

In embodiments of the first aspect of the present invention, the space between the surfaces includes an inert gas or a gas that ions react with.

In embodiments of the first aspect of the present invention, the ion mobility unit further comprises multiple cyclotron stages.

In embodiments of the first aspect of the present invention, the multiple cyclotron stages are arranged in a stack.

In embodiments of the first aspect of the present invention, the ion mobility unit is coupled to at least one of the following: a charge detector, an optical detector, and a mass spectrometer.

In embodiments of the first aspect of the present invention, wherein the ions are introduced from outside the ion mobility unit and formed using at least one of the following: photoionization, Corona discharge, laser ionization, electron impact, field ionization, chemical ionization, and electrospray.

In embodiments of the first aspect of the present invention, the electric field is a static electric field or a dynamic electric field.

In embodiments of the first aspect of the present invention, the static field is a DC gradient and the dynamic field is a traveling wave.

In embodiments of the first aspect of the present invention, the RF potentials, having two or more distinct frequencies and different electric field strengths, are co-applied to the arrays of electrodes on the surfaces and with a pattern of application that creates a pseudopotential that inhibits charged particles from approaching one or both of the surfaces over a substantially greater m/z range than would be feasible with other RF potentials of a single frequency.

In embodiments of the first aspect of the present invention, the surfaces are one of the following: planar, parallel, and not flat. In embodiments of the first aspect of the present invention, the selectivity enhancer station comprises the ion mobility unit and at least one unit selected from the following group: ion transportation unit, ion fragmentation unit.

In embodiments of the first aspect of the present invention, the ion mobility unit is for spatial mobility filtering and/or temporal mobility filtering.

In embodiments of the first aspect of the present invention, the ion transportation unit is selected from the group consisting of ion funnels, step wave, S-lens, ion mirror, ion fight tube by differential potential, ion trap, multipole, e.g. quadrupole, non-contact ion transport system, e.g. direct source coupling in which a capillary (e.g. heated capillary) or two lenses with a electrostatic gradient is a connection for two ion handling units (e.g. source and a quadrupole) and magnetic sector transport.

In embodiments of the first aspect of the present invention, the ion fragmentation unit is selected from the group consisting of collision-induced dissociation (CID), electron-capture dissociation (ECD), electron-transfer dissociation (ETD), photon dissociation and electron impact (EI).

In embodiments of the first aspect of the present invention, the ion mobility unit is arranged between the ion transportation unit and an ion fragmentation unit.

In embodiments of the first aspect of the present invention, at least one selectivity enhancer station comprises or consists of one ion fragmentation unit or two ion fragmentation units or three ion fragmentation units or four ion fragmentation units. The ion mobility unit can be arranged between two ion fragmentation units, preferably the ion mobility unit can directly couplet to a first ion fragmentation unit and directly coupled to a second ion fragmentation unit.

In embodiments of the first aspect of the invention, the analyte of interest pass through a first ion fragmentation unit, then the ion mobility unit, and then a second ion fragmentation unit.

In embodiments of the first aspect of the invention, the analyte of interest pass through the ion transportation unit, then the ion mobility unit, and then a second ion fragmentation unit.

In embodiments of the first aspect of the invention, the first ion fragmentation unit is selected from the group consisting of collision-induced dissociation (CID), electron-capture dissociation (ECD), electron-transfer dissociation (ETD), and photon dissociation, electron impact (EI).

In embodiments of the first aspect of the invention, the second ion fragmentation unit is selected from the group consisting of ion trap, orbitrap, time-of-flight (TOF), magnetic sector, e.g. double focusing sector field (DFS).

In embodiments of the first aspect of the invention, the first ion fragmentation unit is arranged before the ion mobility unit.

In embodiments of the first aspect of the invention, the second ion fragmentation unit is arranged after the ion mobility unit.

In embodiments of the first aspect of the invention, the ion transportation unit is arranged before the ion mobility unit, preferably before the first ion fragmentation unit.

In embodiments of the first aspect of the present invention, the collision-induced dissociation (CID) is related to the collision of the target analyte with a neutral gas (e.g. argon, nitrogen). The target analyte gets the energy for the collion due to a electric field driven velocity.

In embodiments of the first aspect of the present invention, the electron-capture dissociation (ECD) is related to a dissociation driven by electrons from a supporting component which is a odd-electron species.

In embodiments of the first aspect of the present invention, the electron-transfer dissociation (ETD) is related to an electron to be transferred to the positive precursor molecules radical anions are generated and put into the ion trap with them. During the ion/ion reaction an electron is transferred to the positively-charged analyte molecule, causing fragmentation.

In embodiments of the first aspect of the present invention, the photon dissociation is related to the process of storing analyte ions (e.g. in a ion trap) followd by irridation of light with an energy to be capable to break the analyte bonds.

In embodiments of the first aspect of the present invention, the ion trap is related to a device which stores ions based upon their m/z ratio by modulating AC/DC and RF values which are applied to the ion trap cell in which the ions are stored.

In embodiments of the first aspect of the present invention, the time-of-flight (TOF) is related to a time dependend technique in which the ions are accellarated by electric fields with a defined time point for the start. The ions reach the detector after certain time which is directly dependend on their m/z value.

In embodiments of the first aspect of the present invention, the double focusing magnetic sector is related to a combination of an permanent magnet and an electromagnetic magnet which directs the ions through a space and by the two sectors different m/z values can be separated.

The clinical diagnostic system comprises an ion detection station. The ion detection station is for detecting of at least one ion of the at least one analyte of interest.

In embodiments of the first aspect of the present invention, the ion detection station comprises at least one unit or more than one units selected from the group: faraday cup, microchannel plate detector (MCP), photomultiplier, orbitrap, electron multiplier, photographic plate.

In embodiments of the first aspect of the present invention, the faraday cup is related to a metal (conductive) cup designed to catch charged particles in vacuum. The resulting current can be measured and used to determine the number of ions or electrons hitting the cup.

In embodiments of the first aspect of the present invention, the microchannel plate detector (MCP) is related to a planar component used for detection of single particles (electrons, ions and neutrons).

In embodiments of the first aspect of the present invention, the photomultiplier is related to a device for which an analyte molecule hits a first dynode an therefore amplifies the hit process on the first dynode by ejection of multiple other charge units which therefore hits the next dynode etc. In the end a ion sensitive detector can record the multiplied signal.

In embodiments of the first aspect of the present invention, the orbitrap is related to an ion trap mass analyzer consisting of an outer barrel-like electrode and a coaxial inner spindle-like electrode that traps ions in an orbital motion around the spindle. The image current from the trapped ions is detected and converted to a mass spectrum using the Fourier transform of the frequency signal.

In embodiments of the first aspect of the present invention, the electron multiplier is related to a vacuum-tube structure that multiplies incident charges. In a process called secondary emission, a single electron can, when bombarded on secondary-emissive material, induce emission of roughly 1 to 3 electrons. If an electric potential is applied between this metal plate and yet another, the emitted electrons will accelerate to the next metal plate and induce secondary emission of still more electrons. This can be repeated a number of times, resulting in a large shower of electrons all collected by a metal anode, all having been triggered by just one.

In embodiments of the first aspect of the invention, the photographic plate is a ion sensitive plated by a chemical reaction. This plate can fix the ion image after followed chemical reactions (e.g. like a photographic process with silver ions).

In embodiments of the first aspect of the invention, the ion detection station is a mass spectrometer. The ion detection station can perform mass spectrometry.

In embodiments of the first aspect of the invention, the ion detection station is a tandem mass spectrometer.

Most common sample workflows in MS include sample preparation and/or enrichment steps, wherein the analyte(s) of interest are separated from the matrix using gas or liquid chromatography. The inventor surprisingly found that a clinical diagnostic system of the first aspect of the invention, which is preferably free of an chromatography unit, e.g. as part of the selectivity enhancer station, shows advantages compared to a system comprising a chromatography unit. This is mostly performed by removing any parts in which mechanical elements are moved which are in contact to each other (e.g. pumps). The removal of aggressive solvents (e.g. acetonitril) is firstly advantegeous for the environment and secondly it prevents chemically unstable materials (e.g. rubber sealings) from breakedown. The removal of stationary phases for separation is advantouge to reduce overall costs due to clogging and destruction of those stationary phases. The time for changing conditions in traditionally HPLC is very time consuming which gets vanished if only gases or electric fields need to be changed.

In embodiments of the first aspect of the invention, the clinical diagnostic system is performed in the multiple raction mode.

In embodiments of the first aspect of the invention, the ion detection station is a charge detector or an optical detector.

In embodiments of the first aspect of the invention, the charge detector refers to a semiconductor which can recognize a chared molecule by applying a voltage.

In embodiments of the first aspect of the invention, the optical detector refers to a semiconductor which can recognize a photon by applying a voltage.

The clinical diagnostic system comprises a data processing station. The data processing station is for processing and/or evaluation of at least one electronic signal received at least from the ion detection system.

In embodiments of the first aspect of the invention, the data processing station is computer based. Computer based can mean in this context that a computer is used or processing and/or evaluation and/or showing the at least one electronic signal or the information, the user wanted to know.

In embodiments of the first aspect of the invention, the data evaluation, i.e. identification and possibly quantitation and/or qualification of each analyte of interest for each sample can be made in the data processing station.

In embodiments of the first aspect of the invention, the analyte of interest passes through the sample preparation station, then the ion generation station, then the at least one selectivity enhancer station, then the ion detection station and then the data processing station.

In embodiments of the first aspect of the invention, the the analyte of interest passes through the ion transportation unit, then a first ion fragmentation unit, then the ion mobility unit, and then a second ion fragmentation unit.

In embodiments of the first aspect of the invention, the at least one analyte of interest is present in the said clinical diagnostic system and/or in the mass spectrometer and/or in the ion mobility unit of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.

In a second aspect, the present invention relates to the use of the clinical diagnostic system of the first aspect of the present invention for determining the presence or the level of the at least one analyte of interest in the biological sample.

All embodiments mentioned for the first aspect of the invention apply for the second aspect of the invention and vice versa.

In a third aspect, the present invention relates to a method for determining the presence or level of at least one analyte of interest in a biological sample comprising:
A) Preparation of the biological sample, preferably automated preparation of the biological sample comprising at least one analyte of interest,
B) Ion generation from the at least one analyte of interest,
C) Separation the ion or ions via their ion mobility using an electric field applied to one or more electrodes,
D) Detection and determining of at least one ion of the at least one analyte of interest using mass spectrometry.

All embodiments mentioned for the first aspect of the invention and/or second aspect of the invention apply for the third aspect of the invention and vice versa.

In embodiments of the third aspect of the present invention, the internal standard is added, preferably in step (A), wherein the internal standard is preferably isotopically labelled.

In embodiments of the third aspect of the present invention, the analyte of interest is native or not native.

In embodiments of the third aspect of the invention, the method is an in vitro method.

In embodiments of the third aspect of the present invention, the method is performed in a clinical diagnostic system according to the first aspect of the present invention.

In a fourth aspect, the present invention relates to a kit suitable to perform a method of the third aspect of the present invention comprising
- reagents, preferably for performing the pre-treatment,
- magnetic, paramagnetic or supraparamagnetic beads for performing the enrichment, and
- optionally an internal standard, which is preferably isotopically labelled.

All embodiments mentioned for the first aspect of the invention and/or second aspect of the invention and/or third aspect of the invention apply for the fourth aspect of the invention and vice versa.

In embodiments of the fourth aspect of the present invention, the pre-treatment is performed in the sample pre-treatment unit.

In embodiments of the fourth aspect of the present invention, the magnetic or paramagnetic beads are used in the magnetic bead handling unit.

In embodiments of the fourth aspect of the present invention, the internal standard is used in the internal standard handling unit.

In a fifth aspect, the present invention relates to the use of a kit in a clinical diagnostic system of the first aspect of the present invention and/or in a method of the third aspect of the present invention.

All embodiments mentioned for the first aspect of the invention and/or second aspect of the invention and/or third aspect of the invention and/or fourth aspect of the invention apply for the fifth aspect of the invention and vice versa.

In a first aspect, the present invention relates to a clinical diagnostic system comprising
- a sample preparation station for the automated preparation of biological samples comprising at least one analyte of interest,
- an ion generation station for generating at least one ion or ions of the at least one analyte of interest,
- at least one selectivity enhancer station or more for enhancing the selectivity of the at least one analyte of interest, wherein the at least one selectivity enhancer station separates ions based on the respective ion size comprises an ion mobility unit for separation ions with respect to their ion mobility using electric and/or radio frequency fields (AC/DC and/or RF) applied to one or more electrodes,
- an ion detection station for detecting of at least one ion of the at least one analyte of interest, and
- a data processing station for processing and/or evaluation of at least one electronic signal received at least from the ion detection system.

In embodiments of the first aspect of the invention, the biological sample are prepared in a random-excess mode.

In embodiments of the first aspect of the invention, the biological sample is obtained from a patient sample, which is selected from a group consisting of serum, plasma, tissue, liquor, breath, sweat, sputum and whole blood sample from an individual, preferably wherein the individual is a mammal, preferably a human.

In embodiments of the first aspect of the invention, the at least one analyte of interest has a molar mass of smaller than m/z= 20 000, preferably smaller than m/z = 10 000.

In embodiments of the first aspect of the invention, the sample preparation station is for removing or at least reducing interfering matrix components in the biological sample and/or enriching analytes of interest in the biological sample and comprises at least one of the following units or combinations thereof: sample delivery unit, sample pre-treatment unit, sample analytical unit.

In embodiments of the first aspect of the invention, the sample delivery unit comprises sample collection and preparation and/or transportation. According to the first aspect of the invention, the sample pre-treatment unit comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit and/or the sample analytical unit may comprise at least one unit or more than one units selected from the group: a solid-liquid support analyte enrichment unit, solid-liquid support matrix depletion unit, gaseous sample enrichment or matrix separation unit, solid sample on solid support unit.

In embodiments of the first aspect of the invention, the sample preparation station is partially or fully automated.

In embodiments of the first aspect of the invention, the ion generation station comprises an unsteady analyte ion supply unit and/or a steady analyte ion supply unit, wherein the unsteady analyte ion supply unit is selected from the group consisting of laser desorption ionzation (LDI), dielectric barrier liquid flow, surface plasma ionization and combinations thereof, wherein the steady analyte ion supply unit is selected from the group consisting of flow injection (preferably flow injection using ESI or APCI or Nano Spray), paper spray, electron ionization (EI), matrix assisted ionization (MAI), chemical ionization (CI), e.g. proton-transfer-reaction (PTR), radioactive supported ionization, e.g. using ⁶³Ni and combinations thereof.

In embodiments of the first aspect of the invention, the selectivity enhancer station comprises the ion mobility unit and at least one unit selected from the following group: ion transportation unit, ion fragmentation unit, wherein the ion transportation unit is selected from the group consisting of ion funnels, step wave, S-lens, ion mirror, ion fight tube by differential potential, ion trap, multipole, e.g. quadrupole, non-contact ion transport system, e.g. direct source coupling (e.g. heated capillary) and magnetic sector transport, wherein the ion fragmentation unit is selected from the group consisting of collision-induced dissociation (CID), electron-capture dissociation (ECD), electron-transfer dissociation (ETD), photon dissociation, ion trap, orbitrap, time-of-flight (TOF), magnetic sector, e.g. double focussion sector field (DFS) and combinations thereof.

In embodiments of the first aspect of the invention, the at least one analyte of interest is present in the said clinical diagnostic system of less than 10 seconds, preferably less than 9 seconds or 8 seconds or 7 seconds or 6 seconds or 5 seconds or 4 seconds or 4 seconds or 3 seconds or 2 seconds or 1 second.

In embodiments of the first aspect of the invention, the clinical diagnostic system is free of paper spray or Solid Phase Extraction (SPE). Preferably, the ion generation station for generating at least one ion or ions of the at least one analyte of interest is free of or does not comprise paper spray or SPE.

In embodiments of the first aspect of the invention, the clinical diagnostic system is free of an ion mobility separator comprising at least one coaxial mobility cell followed by conical coaxial ion channel.

In embodiments of the first aspect of the present invention, the ion mobility unit comprises a printed circuit board, preferably a stack of printed circuit boards.

In embodiments of the first aspect of the present invention, the ion mobility unit is free of a printed circuit board, preferably free of a stack of printed circuit boards.

In embodiments of the first aspect of the present invention, the clinical diagnostic system does not comprise an ion source, preferably said source being filled with gas at gas pressure from generally at or between about 1 mBar to 1Bar;
an ion gate formed of a front cap electrode, followed by a front mesh and then by a back mesh, wherein the meshes are generally parallel with one another and spaced at a distance comparable to mesh cell size;
a radiofrequency (RF) generator connected between the meshes;
a switching or adjustable DC signal connected to the cap electrode and meshes;
an ion drift space, preferably filled with gas at pressure from generally at or between about (1 to 30) mBar; and an ion detector.

In embodiments of the first aspect of the invention, the clinical diagnostic system is free of gas chromatography (GC).

In embodiments of the first aspect of the present invention, the clinical diagnostic system does not separate positively charged ions and negatively charged ions in the gas phase.

In embodiments of the first aspect of the present invention, the clinical diagnostic system does not comprise an annular electrode.

In embodiments of the first aspect of the present invention, the clinical diagnostic system does not comprise an ion drift space, that preferably is filled with gas at pressure from substantially at or between about (1 to 100) mBar.

In embodiments of the first aspect of the present invention, the clinical diagnostic system does not comprise an ion drift tube.

In a second aspect, the present invention relates to the use of the clinical diagnostic system of the first aspect of the present invention for determining the presence or the level of the at least one analyte of interest in the biological sample.

In a third aspect, the present invention relates to a method for determining the presence or level of at least one analyte of interest in a biological sample comprising:
A) Preparation of the biological sample, preferably automated preparation of the biological sample comprising at least one analyte of interest,
B) Ion generation from the at least one analyte of interest,
C) Separation the ion or ions via their ion mobility using an electric field applied to one or more electrodes,
D) Detection and determining of at least one ion of the at least one analyte of interest using mass spectrometry.

In embodiments of the third aspect of the invention, the internal standard is added, preferably in step (A), wherein the internal standard is preferably isotopically labelled.

In embodiments of the third aspect of the invention, the said method is performed in a clinical diagnostic system according to the first aspect of the present invention.

In a fourth aspect, the present invention relates to a kit suitable to perform a method of the third aspect of the invention comprising
- reagents, preferably for performing the pre-treatment,
- magnetic, paramagnetic or supraparamagnetic beads for performing the enrichment, and
- optionally an internal standard, which is preferably isotopically labelled.

In a fifth aspect, the present invention relates to the use of a kit in a clinical diagnostic system of the first aspect of the invention and/or in a method of third aspect of the invention.

In further embodiments, the present invention relates to the following aspects:
1. A clinical diagnostic system comprising
   - a sample preparation station for the automated preparation of biological samples comprising at least one analyte of interest,
   - an ion generation station for generating at least one ion or ions of the at least one analyte of interest,
   - at least one selectivity enhancer station or more for enhancing the selectivity of the at least one analyte of interest, wherein the at least one selectivity enhancer station separates ions based on the respective ion size comprises an ion mobility unit for separation ions with respect to their ion mobility using electric and/or radio frequency fields (AC/DC and/or RF) applied to one or more electrodes,
   - an ion detection station for detecting of at least one ion of the at least one analyte of interest, and
   - a data processing station for processing and/or evaluation of at least one electronic signal received at least from the ion detection system.
2. The clinical diagnostic system of aspect 1, wherein the said clinical diagnostic system is for determining the presence or the level of at least one analyte of interest.
3. The clinical diagnostic system of aspect 1 or 2, wherein the sample preparation station, the ion generation station, the at least one selectivity enhancer station, the ion detection station and the data processing station are connected to each other.
4. The clinical diagnostic system of any of the proceeding aspects, wherein the biological sample are prepared in a random-excess mode and/or wherein the clinical diagnostic system is automated.
5. The clinical diagnostic system of any of the proceeding aspects, wherein the ion or ions of the at least one analyte of interest are in the gaseous state.
6. The clinical diagnostic system of any of the proceeding aspects, wherein the at least one selectivity enhancer station comprises
   - at least one ion storage unit and/or ion transportation unit with no ion separation capability , and
   - an ion selection unit which separates ions based on their m/z (mass over charge ratio).
7. The clinical diagnostic system of any of the proceeding aspects, wherein the biological sample is obtained from a patient sample, which is selected from a group consisting of serum, plasma, tissue, liquor, breath, sweat, sputum and whole blood sample from an individual.
8. The clinical diagnostic system of any of the proceeding aspects, wherein the individual is a mammal, preferably a human.
9. The clinical diagnostic system of any of the proceeding aspects, wherein the at least one analyte of interest is a small molecule.
10. The clinical diagnostic system of any of the proceeding aspects, wherein the at least one analyte of interest has a molar mass of smaller than m/z= 2000 , preferably smaller than m/z =1000 .
11. The clinical diagnostic system of any of the proceeding aspects, wherein the at least one analyte of interest is not a large molecule having a m/z of < 2000, preferably the at least one analyte of interest is selected from the following group: testosterone, vitamin D, cyclosporine A, lidocaine, sirolimus, abeta 42.
12. The clinical diagnostic system of any of the proceeding aspects, wherein the at least one analyte of interest is selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.
13. The clinical diagnostic system of any of the proceeding aspects, wherein the biological samples comprises the at least one analyte of interest and a matrix, wherein preferably the matrix comprises the following components or combinations thereof: inorganic salt, organic salt component, protein, solvents polymer, small molecule.
14. The clinical diagnostic system of any of the proceeding aspects, wherein the matrix is separated at least in the sample preparation station from the at least one analyte of interest.
15. The clinical diagnostic system of any of the proceeding aspects, wherein the sample preparation station comprises at least one of the following units or combinations thereof: sample delivery unit, sample pre-treatment unit, sample analytical unit.
16. The clinical diagnostic system of any of the proceeding aspects, wherein the sample preparation station is for removing or at least reducing interfering matrix components in the biological sample and/or enriching analytes of interest in the biological sample.
17. The clinical diagnostic system of any of the proceeding aspects, wherein the sample delivery unit comprises sample collection and preparation and/or transportation.
18. The clinical diagnostic system of any of the proceeding aspects, wherein the sample pre-treatment unit comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit.
19. The clinical diagnostic system of any of the proceeding aspects, wherein the sample preparation station is partially or fully automated and/or wherein the clinical diagnostic system is partially or fully automated.
20. The clinical diagnostic system of any of the proceeding aspects, wherein the one or more pipetting units and/or fluid transport units are for adding, mixing and/or removing fluids.
21. The clinical diagnostic system of any of the proceeding aspects, wherein the reaction container transporting mechanism unit is for delivering sample or to the following process station.
22. The clinical diagnostic system of any of the proceeding aspects, wherein the magnetic bead handling unit is for treating the biological sample with magnetic beads carrying analyte and/or matrix selective groups.
23. The clinical diagnostic system of any of the proceeding aspects, wherein the internal standard handling unit is for the addition of at least one internal standard and/or standard addition.
24. The clinical diagnostic system of any of the proceeding aspects, wherein the supporting compound handling unit is for the addition of supporting compounds or ion supporting molecules, e.g. in order to release the at least one analyte of interest from a binder, e.g. methanol or NaOH, or from a matrix or in order to add a matrix, e.g. 2,5-Dihydroxybenzoesäure (DHB) for MALDI applications or treat the analyte of interest with a respective derivatization agent to change the chemical and/or physical properties of the analyte and/or the matrix.
25. The clinical diagnostic system of any of the proceeding aspects, wherein the sample analytical unit comprises at least one unit or more than one units selected from the group: a solid-liquid support analyte enrichment unit, solid-liquid support matrix depletion unit, gaseous sample enrichment or matrix separation unit, solid sample on solid support unit.
26. The clinical diagnostic system of any of the proceeding aspects, wherein the solid-liquid support analyte enrichment unit is selected form the group consisting of solid-phase extraction (SPE), micro particle, liquid-liquid extraction (LLE), liquid chromatography (LC) and high pressure liquid chromatography (HPLC).
27. The clinical diagnostic system of any of the proceeding aspects, wherein the solid-liquid support matrix depletion unit is selected form the group consisting of solid-phase extraction (SPE), micro particle and liquid-liquid extraction (LLE) or combinations thereof.
28. The clinical diagnostic system of any of the proceeding aspects, wherein the gaseous sample enrichment or matrix separation unit is inert gas stripping or headspace extraction or gas adsorption supported by liquid and/or solid e.g. activated charcoal or combinations thereof.
29. The clinical diagnostic system of any of the proceeding aspects, wherein the solid sample on solid support unit is imaging of cross sections and mineral analysis.
30. The clinical diagnostic system of any of the proceeding aspects, wherein the ion generation station comprises an unsteady analyte ion supply unit and/or a steady analyte ion supply unit.
31. The clinical diagnostic system of any of the proceeding aspects, wherein the unsteady analyte ion supply unit is selected from the group consisting of laser desorption ionzation (LDI), dielectric barrier liquid flow, surface plasma ionization and combinations thereof.
32. The clinical diagnostic system of any of the proceeding aspects, wherein the steady analyte ion supply unit is selected from the group consisting of flow injection (preferably flow injection using ESI or APCI or Nano Spray), paper spray, electron ionization (EI), matrix assisted ionization (MAI), chemical ionization (CI), e.g. proton-transfer-reaction (PTR), radioactive supported ionization, e.g. using ⁶³Ni and combinations thereof.
33. The clinical diagnostic system of any of the proceeding aspects, wherein the selectivity enhancer station comprises the ion mobility unit and at least one unit selected from the following group: ion transportation unit, ion fragmentation unit.
34. The clinical diagnostic system of any of the proceeding aspects, wherein the ion mobility unit is for spatial mobility filtering and/or temporal mobility filtering.
35. The clinical diagnostic system of any of the proceeding aspects, wherein the ion transportation unit is selected from the group consisting of ion funnels, step wave, S-lens, ion mirror, ion fight tube by differential potential, ion trap, multipole, e.g. quadrupole, non-contact ion transport system, e.g. direct source coupling (e.g. heated capillary) and magnetic sector transport.
36. The clinical diagnostic system of any of the proceeding aspects, wherein the ion fragmentation unit is selected from the group consisting of collision-induced dissociation (CID), electron-capture dissociation (ECD), electron-transfer dissociation (ETD), photon dissociation, ion trap, orbitrap, time-of-flight (TOF), magnetic sector, e.g. double focussion sector field (DFS) and combinations thereof.
37. The clinical diagnostic system of any of the proceeding aspects, wherein the ion detection station comprises at least one unit or more than one units selected from the group: faraday cup, microchannel plate detector (MCP), photomultiplier, orbitrap, electron multiplier, photographic plate.
38. The clinical diagnostic system of any of the proceeding aspects, wherein the ion detection station is a mass spectrometer.
39. The clinical diagnostic system of any of the proceeding aspects, wherein the ion detection station is a charge detector or an optical detector.
40. The clinical diagnostic system of any of the proceeding aspects, wherein the data processing station is computer based.
41. The clinical diagnostic system of any of the proceeding aspects, wherein the data processing station performs the calculation of the results from the electronic signal.
42. The clinical diagnostic system of any of the proceeding aspects, wherein the analyte of interest pass through the sample preparation station, then the ion generation station, then the at least one selectivity enhancer station, then the ion detection station and then the data processing station.
43. The clinical diagnostic system of any of the proceeding aspects, wherein said clinical diagnostic system comprises more than one the selectivity enhancer stations, e.g. 2 or 3 or 4 or 5 or 6.
44. The clinical diagnostic system of any of the proceeding aspects, wherein said clinical diagnostic system comprises exactly one selectivity enhancer station.
45. The clinical diagnostic system of any of the proceeding aspects, wherein the analyte of interest pass through the ion transportation unit, then a first ion fragmentation unit, then the ion mobility unit, and then a second ion fragmentation unit.
46. The clinical diagnostic system of any of the proceeding aspects, wherein the analyte of interest pass through a first ion fragmentation unit, then the ion mobility unit, and then a second ion fragmentation unit.
47. The clinical diagnostic system of any of the proceeding aspects, wherein the analyte of interest pass through the ion transportation unit, then the ion mobility unit, and then a second ion fragmentation unit.
48. The clinical diagnostic system of any of the proceeding aspects, wherein the analyte of interest pass through the ion transportation unit, then a first ion fragmentation unit, and then the ion mobility unit.
49. The clinical diagnostic system of any of the proceeding aspects, wherein the first ion fragmentation unit is selected from the group consisting of collision-induced dissociation (CID), electron-capture dissociation (ECD), electron-transfer dissociation (ETD), photon dissociation and combinations thereof.
50. The clinical diagnostic system of any of the proceeding aspects, wherein the second ion fragmentation unit is selected from the group consisting of ion trap, orbitrap, time-of-flight (TOF), magnetic sector, e.g. double focusing sector field (DFS) and combinations thereof.
51. The clinical diagnostic system of any of the proceeding aspects, wherein the first ion fragmentation unit is arranged before the ion mobility unit.
52. The clinical diagnostic system of any of the proceeding aspects, wherein the at least one analyte of interest is present in the said clinical diagnostic system or in the mass spectrometer or in the ion mobility unit of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.
53. The clinical diagnostic system of any of the proceeding aspects, wherein the second ion fragmentation unit is arranged after the ion mobility unit.
54. The clinical diagnostic system of any of the proceeding aspects, wherein the ion transportation unit is arranged before the ion mobility unit, preferably before the first ion fragmentation unit.
55. The clinical diagnostic system of any of the proceeding aspects, wherein the ions with similar or equal ion mobility unit bunch together in the ion mobility unit.
56. The clinical diagnostic system of any of the proceeding aspects, wherein the ion mobility unit performs a gas phase separation of a mixture of ions.
57. The clinical diagnostic system of any of the proceeding aspects, wherein the ions have a mass to charge ratio the range of 1 to about 100,000 in the ion mobility unit.
58. The clinical diagnostic system of any of the proceeding aspects, wherein the ions have a drift time through the ion mobility unit of about 0 to about 60 seconds.
59. The clinical diagnostic system of any of the proceeding aspects, wherein at least a portion of the ion mobility unit is maintained at a pressure in the range of about 10-3 torr to atmospheric pressure.
60. The clinical diagnostic system of any of the proceeding aspects, wherein the ions in the ion mobility unit are formed by using at least one of the following: photoionization, Corona discharge, laser ionization, electron impact, field ionization, chemical ionization, and electrospray.
61. The clinical diagnostic system of any of the proceeding aspects, wherein the ions are formed outside of the ion mobility unit, preferably in the ion generation station.
62. The clinical diagnostic system of any of the proceeding aspects, wherein the ion mobility unit comprises a non-constant electric field applied to the ion mobility unit to form a potential gradient along a portion of the ion mobility unit so that ions with similar mobilities bunch together into sharper peaks while maintaining separation between other ions.
63. The clinical diagnostic system of any of the proceeding aspects, wherein the potential gradient is a DC gradient.
64. The clinical diagnostic system of any of the proceeding aspects, wherein the potential gradient progressively increases or decreases along the length of the device.
65. The clinical diagnostic system of any of the proceeding aspects, wherein the potential gradient along a length of the device is between 0 to about 5,000 volts/mm.
66. Use of the clinical diagnostic system of any one of aspects 1 to 65 for determining the presence or the level of the at least one analyte of interest in the biological sample.
67. A method for determining the presence or level of at least one analyte of interest in a biological sample comprising:
   A) Preparation of the biological sample, preferably automated preparation of the biological sample comprising at least one analyte of interest,
   B) Ion generation from the at least one analyte of interest,
   C) Separation the ion or ions via their ion mobility using an electric field applied to one or more electrodes,
   D) Detection and determining of at least one ion of the at least one analyte of interest using mass spectrometry.
68. The method of aspect 67, wherein the internal standard is added, preferably in step (A), wherein the internal standard is preferably isotopically labelled.
69. The method of aspect 67 or 68, wherein said method is computer-implemented.
70. The method of aspect 67 or 68 or 69, wherein the said method is performed in a clinical diagnostic system according to the aspects 1 to 65.
71. A kit suitable to perform a method of any one of aspects 67 to 70 comprising
   - reagents, preferably for performing the pre-treatment,
   - magnetic, paramagnetic or supraparamagnetic beads for performing the enrichment, and
   - optionally an internal standard, which is preferably isotopically labelled.
72. Use of a kit in a clinical diagnostic system of any one of aspects 1 to 65 and/or in a method of any one of aspects 67 to 70.

### Examples

The following examples are provided to illustrate, but not to limit the presently claimed invention.

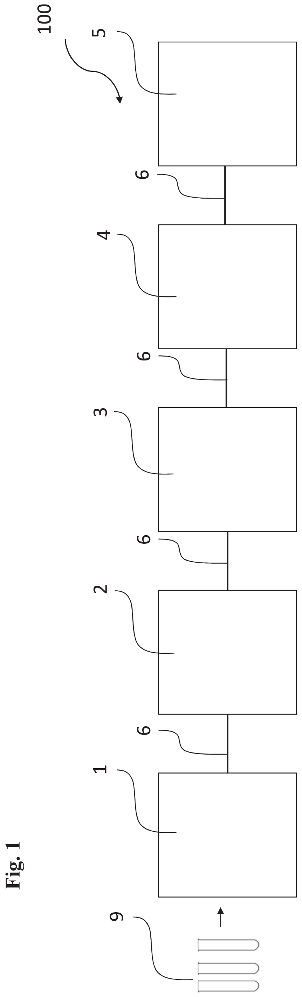

**Figure 1** shows an example of a schematic representation of a clinical diagnostic system 100 and a clinical diagnostic method. The clinical diagnostic system 100 comprises a sample preparation station 1 for the automated preparation of biological samples 9 comprising at least one analyte of interest. The sample preparation station 1 is directly coupled 6 to the ion generation station 2 for generating at least one ion or ions of the at least one analyte of interest. The ion generation station 2 is directly coupled 6 to the selectivity enhancer station 3. The selectivity enhancer station 3 is for enhancing the selectivity of the at least one analyte of interest. The selectivity enhancer station 3 separates ions based on the respective ion size comprises an ion mobility unit 31 for separation ions with respect to their ion mobility using electric field and/or radio frequency fields (AC/DC and/or RF) applied to one or more electrodes. The selectivity enhancer station 3 is directly coupled 6 to the ion detection station 4 for detecting of at least one ion of the at least one analyte of interest. The ion detection station 4 is directly coupled 6 to the data processing station 5 for processing and/or evaluation of at least one electronic signal received at least from the ion detection station 4.

Preferably, the clinical diagnostic system 100 is automated and/or in a random-access mode.

Peferably, the clinical diagnostic system 100 is arranged according to the following embodiments E:

**Table 1:**

| E | Arrangement of the clinical diagnostic system 100 |
|---|---|
| E1 | 11,12,122,131,221,32,31,331,327,46,5 |
| E2 | 11, 12,122,132,221,32,31,331,327,46,5 |
| E3 | 11,12,122,123,134,128,211,31,321,331,324,337,42,5 |
| E4 | 11,12,122,123,134,128,224,31,321,331,324,337,42,5 |
| E5 | 11,12,133,223,31,321,331,324,337,45,5 |
| E6 | 11,12,122,121,222,323,333,326,31,327,42,5 |
| E7 | 11,12,122,128,213,323,326,328,331,328,31,326,331,41,5 |

| | |
|---|---|
| E - embodiment, e.g. E1 - embodiment 1 | |

The numbers mentioned in the table 1 represent the references of the respective stations and/or units mentioned in the list of references.

The numbers are separated by commas, which means that adjacent numbers separating by commas are adjacent stations and/or units, which are coupled. For example:

### embodiment 1 (E1)

The clinical diagnostic system 100 of embodiment 1 (E1) has the following arrangement:
11,12,122,131,221,32,31,331,327,46,5

The sample delivery unit 11, then sample pre-treatment unit 12, then internal standard handling unit 122, then solid-liquid support analyte enrichment unit 131, then flow injection (preferably flow injection using ESI or APCI or Nano Spray) 221, then ion transportation unit 32, then ion mobility unit 31, then collision-induced dissociation (CID) 331, then multipole, e.g. quadrupole 327, then electron multiplier 46 and then data processing station 5.

The advantage of E1 and/or E2 is that the separation of the analytes and therefore the matrix reduction and isobaric separation not only performed by the workflow itself (e.g. magnetic microparticles and or liquid extraction). The ion mobility unit 31 performs as a selectivity enhancer. The task for this enhancer has been made in previously comparable workflows (e.g. with HPLC for isobaric separation and matrix reduction) was done. Furtheremore to add the ion mobility unit 31 in these workflows and substitute HPLC is furthermore advantageous due to the fact that the ion mobility unit 31 will compress the analyte signal time dependently into one peak-shaped signal and therefore further enriches the analyte time dependend.

The advantage of E3 and/or E4 is that for a solid supported analysis from a spot on a surface, when an HPLC separation is not applicale and therefore the ion mobility unit is adding value due to the possibility of having isobaric separation and matrix depletion in ion gas phase.

The advantage of E5 is that the temperature dependend separation of GC (gas chromatography) can be vanished by having the ion mobility unit 31. Therefore no oven for heating or cooling is required to perform the isobaric separation of unwanted matrix components.

The advantage of E6 is that a direct sample analysis on a paper tissue by paper spray ionization can vanish any active zones for matrix reduction or analyte enrichment on the respective paper strip. The separation will be performed therefore with the ion mobility unit 31.

The advantage of E7 is that a surface ionization with a plasma ionization can be used directly on a tissue or a living organism. This surface ionization can be used directly as a cold plasma which not damages living system but ionizes softly the surface molecules which can be further analyzed in real time by mass spectrometry e.g. during a surgery or on cell suspensions.

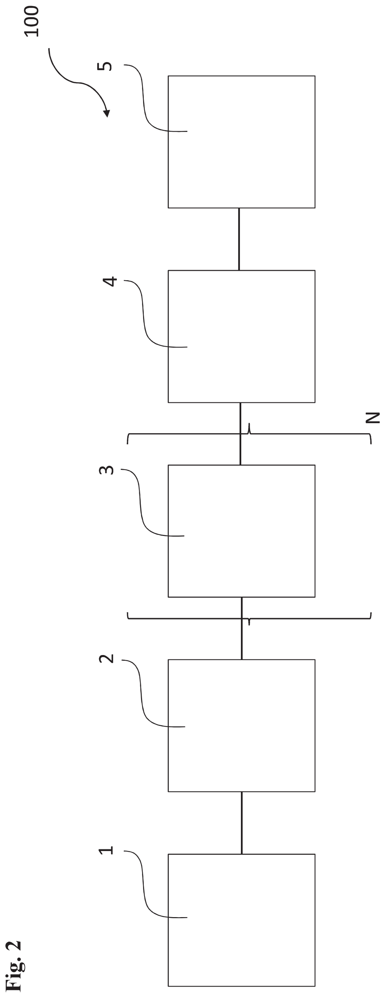

**Figure 2** shows an example of a schematic representation of a clinical diagnostic system 100 and a clinical diagnostic method. In this example of Figure 2 it is shown compared to the clinical diagnostic system 100 of Figure 1 that the selectivity enhancer station 3 can be N-times part of the clinical diagnostic system 100, where N is e.g. 2 or 3 or 4 or 5 or 6. Thus, the enhancement of the selectivity of the at least one analyte of interest can be increased. The advantage of this system is the combination of different fragmentation mechanisms e.g. CID with ETD and in between selection of certain fragments by their respective ion mobility. The selectivity of the system is therefore enhanced if multiple selectivity stations are made through.

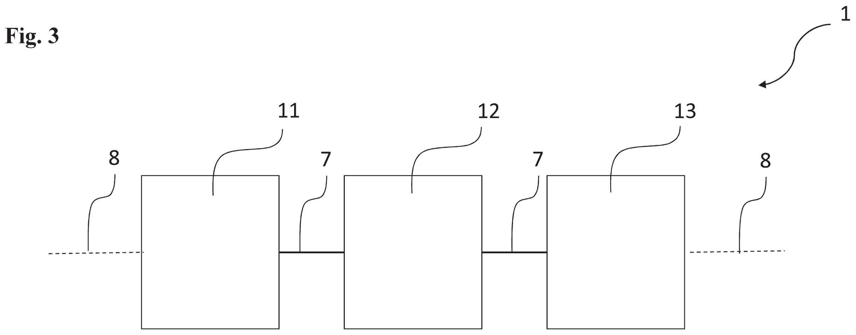

**Figure 3** shows an example of a schematic representation of a sample preparation station 1. The sample preparation station 1 comprises a sample delivery unit 11, a sample pre-treatment unit 12 and a sample analytical unit 13. The sample pre-treatment unit 12 is arranged between the sample delivery unit 11 and the sample analytical unit 13. The sample analytical unit 13 and optionally the sample preparation station 1 can be coupled to adjacent stations and/or units 8. The sample delivery unit 11 comprises sample collection and preparation, e.g. including sample data preparation and/or transportation. In this station the sample is prepared to get addressed by a pipettor e.g. arrended horizontically etc. followed by meta data reading (e.g. barcode reader) followd by sorting if necessary as well as heating or cooling or shaking. The sample pre-treatment unit 12 is for preparing the analyte for quantitative analyses e.g. pipetting of internal standard or adjustment of the pH value. Further binding protein or protein precipitation can be achieved by adding supporting reagents. A heating/cooling or evaporation step can be performed in the sample preparation unit. The sample pre-treatment unit 12 comprises at least one unit or more than one units selected from the following group: magnetic bead handling unit 121, internal standard handling unit 122, supporting compound handling unit 123, pipetting unit 124, fluid transport unit 125, reaction container transporting mechanism unit 126, enrichment handling unit 127, solvent evaporation unit 128. Adjacent to the sample pre-treatment unit 12, the sample analytical unit 13 is arranged. The sample analytical unit 13 is for generation of an ion from the target molecule of interest (analyte). Further the separation of this analyte from unwanted matrix and enhancement of the selectivity by either m/z values or ion mobility. The detection of the selectively enhanced analyte is done by detection of the ion. The sample analytical unit 13 comprises at least one unit or more than one units or a combination of the units selected from the group: a solid-liquid support analyte enrichment unit 131, solid-liquid support matrix depletion unit 132, gaseous sample enrichment or matrix separation unit 133, solid sample on solid support unit 134.

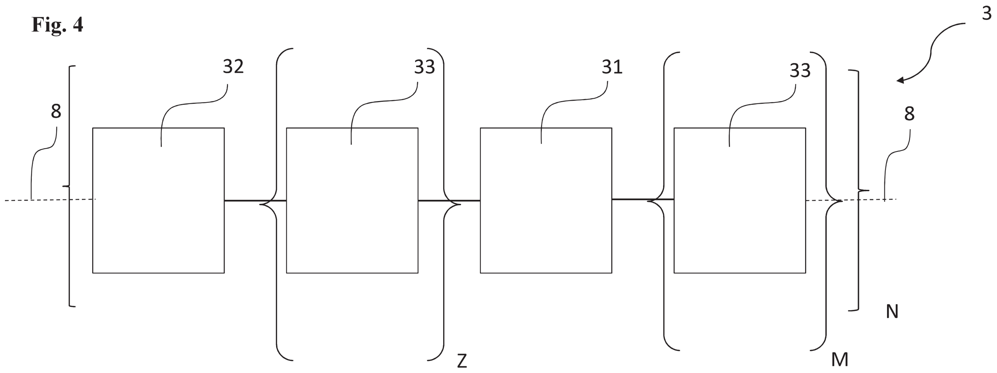

**Figure 4** shows an example of a schematic representation of at least one selectivity enhancer station 3. If N = 1, it is exactly one selectivity enhancer station 3 shown. If N = 2 or 3, then two or three selectivity enhancer stations are examplied shown. The selectivity enhancer stations may have the same or different units and are coupled adjacent to each other. The at least one selectivity enhancer station 3 separates ions based on the respective ion size. The at least one selectivity enhancer station comprises an ion mobility unit 31 for separation ions with respect to their ion mobility using electric and/or radio frequency fields (AC/DC and/or RF) applied to one or more electrodes. The ion mobility unit 31 is arranged directly between two ion fragmentation units 33 (if M and Z > 0) or directly between the ion transportation unit 32 (if Z = 0) and the ion detection station 4 (if M = 0). The ion transportation unit 32 is selected from the group consisting of ion funnels, step wave, S-lens, ion mirror, ion fight tube by differential potential, ion trap, multipole, e.g. quadrupole, non-contact ion transport system, and magnetic sector transport. The ion fragmentation unit 33 is selected from the group consisting of collision-induced dissociation (CID), electron-capture dissociation (ECD), electron-transfer dissociation (ETD), photon dissociation, ion trap, orbitrap, time-of-flight (TOF), magnetic sector, e.g. double focussion sector field (DFS).

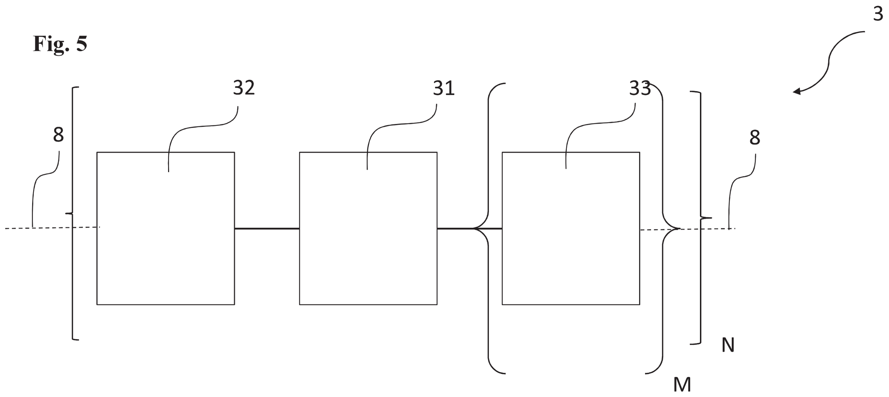

**Figure 5** shows an example of a schematic representation of at least one selectivity enhancer station 3. Here, the ion mobility unit 31 is arranged directly between the ion transportation unit 32 and at least one ion fragmentation unit 33 (if M > 0) or ion detection station 8, 4 (if M = 0).

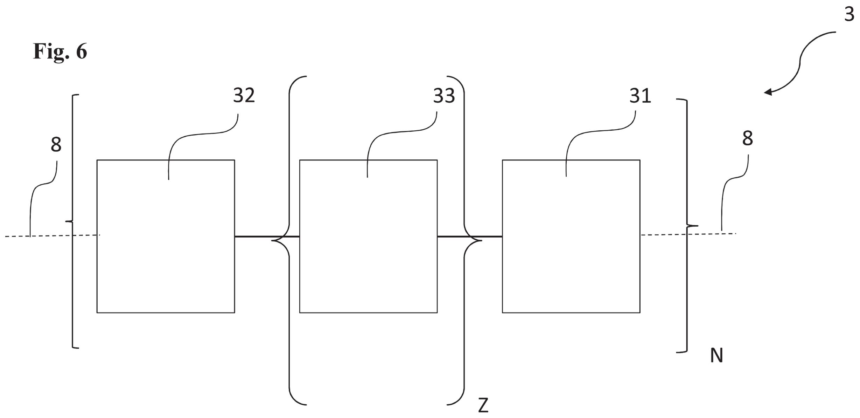

**Figure 6** shows an example of a schematic representation of at least one selectivity enhancer station 3. Here, the ion mobility unit 31 is arranged directly between the ion transportation unit 32 (if Z = 0) or at least one ion fragmentation unit 33 (if Z > 0) and the ion detection station 8, 4. The at least one ion fragmentation unit 33 can be arranged between the ion mobility unit 31 and the ion transportation unit 32.

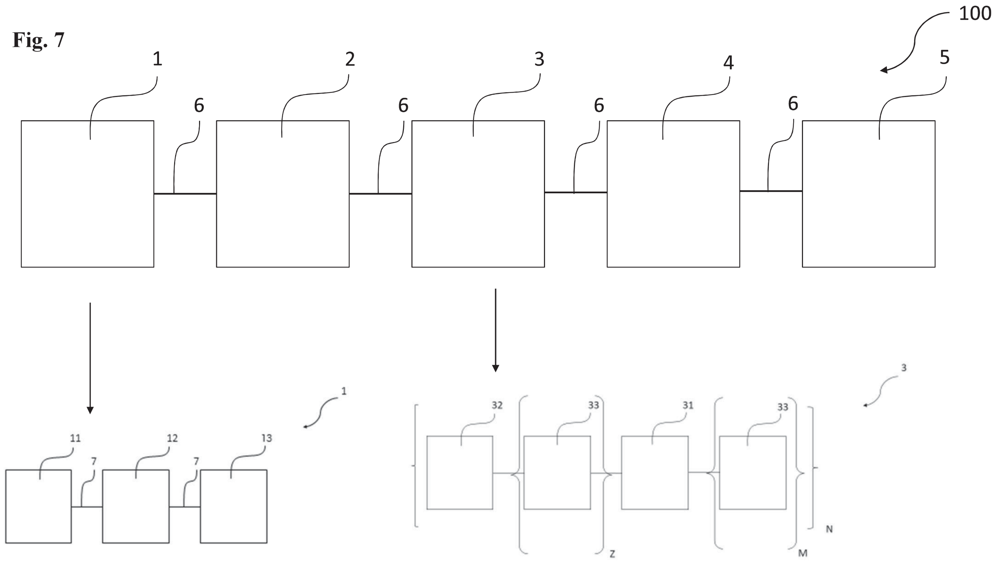

**Figure 7** shows an example of a schematic representation of a clinical diagnostic system 100 and a clinical diagnostic method. This clinical diagnostic system 100 of Figure 7 shows in comparision to the clinical diagnostic system 100 of Figure 1 the units of the sample preparation station 1 (sample delivery unit 11, sample pre-treatment unit 12, sample analytical unit 13) and the units of the selectivity enhancer station 3 as explained in Figure 4.

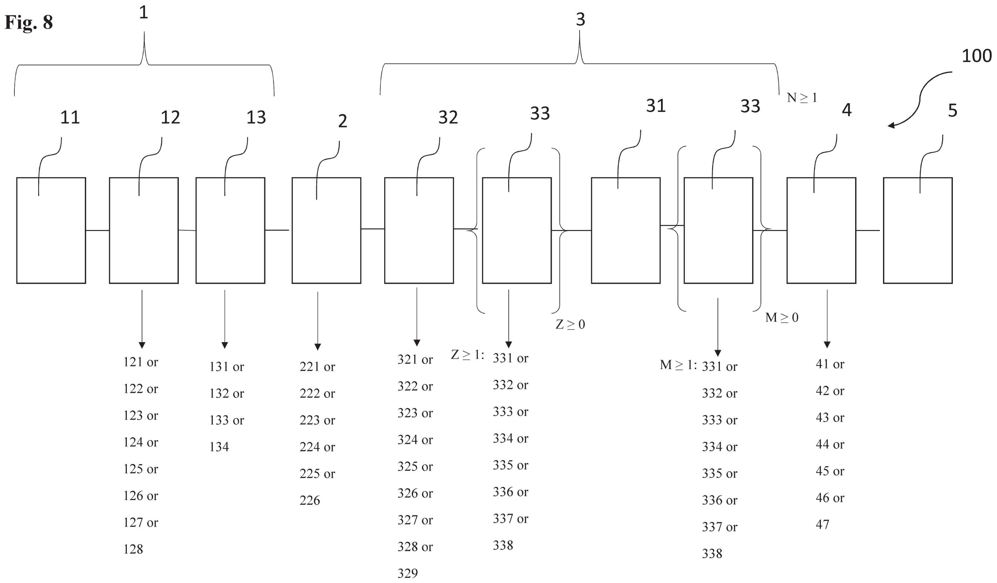

**Figure 8** shows an example of a schematic representation of a clinical diagnostic system 100 and a clinical diagnostic method. Here, the arrangement of the respective stations and units is shown.

Preferably, the following embodiments A to C a clinical diagnostic system 100 are described:
**A) Direct ionization of the sample after sample preparation with nano ESI**
   The clinical diagnostic system 100 consists of or comprises the following units and/or stations: 11,12,121,122,123, 124,13,221,323,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step the analyte loaded magnetic particles gets eluted by a supporting liquid followed by a direct nano-ESI ionization and ion transport by a S-lens to the ion mobility unit which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results.
   The time for the outside of the mass spectrometer is in this workflow about 10s-1000s and inside the mass spectrometer about 0.1s-10s.
**B) Ionization via matrix assisted laser ionization**
   The clinical diagnostic system 100 consists of or comprises the following units and/or stations: 11,12,121,122,123,124,128,134,211,322,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step, the analyte loaded magnetic particles gets eluted by a supporting liquid followed by pipetting and drying onto a LDI target followed by laser irradiation and guiding into the mass spectrometer via an ion funnel to the ion mobility unit which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results.
   The time for the outside of the mass spectrometer is in this workflow about 10s-1000s and inside the mass spectrometer about 0.1s-10s.
**C) Ionization via matrix assited ionization**
   The clinical diagnostic system 100 consists of or comprises the following units and/or stations: 11,12,121,122,123,124,128,134,224,328,31,322,327,331,327,43,5
   The sample enters the workflow by being a human body fluid which is further processed by pipetting internal standard, followed by a magnetic bead workflow, multiple solid liquid separation and washing steps supported by magnetic forces. After the final washing step the analyte loaded magnetic particles gets eluted by a supporting liquid and addition of a supporting solid e.g. matrix compound followed by pipetting and drying onto a glass target followed by non-contact ion transport system (328, direct ion contacting unit) e.g. heated capillary and guiding into the mass spectrometer which separats isobaric components off and passes through the analyte of interest which gets m/z separated by the first quadrupole, fragmented by CID and the fragment separated by m/z with a second quadrupole. An ion detector detects the analyte and the internal standard signal and processes those signals with a computer to finally end with absolute quantitative results.
   The time for the outside of the mass spectrometer is in this workflow about 10s-1000s and inside the mass spectrometer about 0.1s-10s.

With continued reference to Figure 1 a clinical diagnostic method is also illustrated. The method comprises automatically preparing biological samples 9 comprising analytes of interest and inputting prepared samples into ion generation station 2 for generating at least one ion or ions of the at least one analyte of interest, which are gaseous. Then, the ion or ions are separated via their ion mobility using an electric field applied to one or more electrodes in the ion mobility unit 31. Then, the at least one ion of the at least one analyte of interest is detected or determined using mass spectrometry. Peferably the clinical diagnostic method and the clinical diagnostic system 100 is free of a chromatography unit, preferably a liquid chromatography (LC) unit or a HPLC unit.

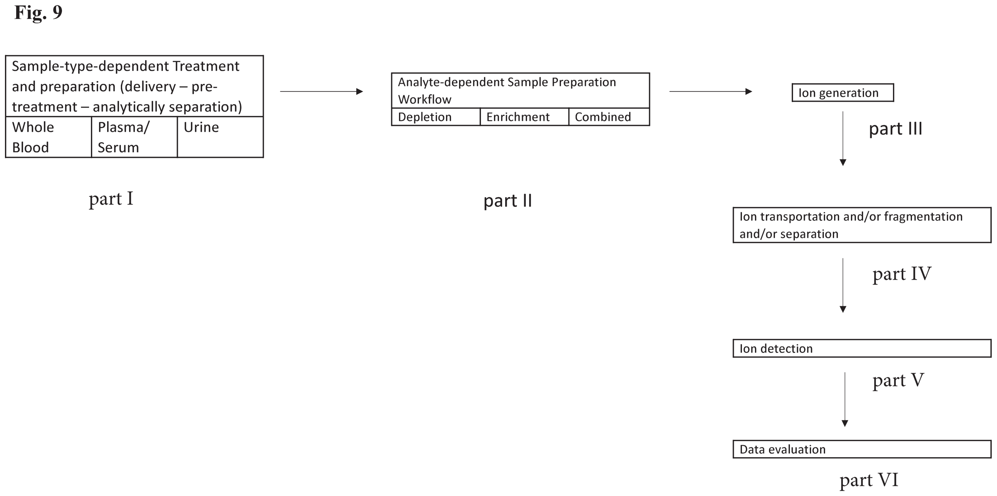

**Figure 9** schematically represents elements of a clinical diagnostic method and in particular a combination of possible workflow paths, depending on the sample type and/or the analytes of interest in a sample.

For example, depending on the type of sample, e.g. whole blood, plasma, serum, urine, a sample can be assigned to one of some pre-defined sample type-dependent pre-treatment workflows (part I) that is most appropriate for that type of sample.

Also, depending on the analytes of interest, e.g. individual analytes or classes of analytes having similar chemical structure or properties, following the sample-type dependent (pre-)treatment in part I, a sample can be assigned to one of some pre-defined analyte-dependent sample preparation workflows, e.g. based on a matrix depletion method, an analyte enrichment method, or a combination of both, that is most appropriate for that type of analyte(s) (part II).

The analytes of interest, a prepared sample can be assigned to a particular ion generation station 2 for generation ions, preferably in gaseous form (part III).

Also, depending on the analytes of interest, a prepared sample can be assigned to a particular selectivity enhancer station 3 for separating or transferring that type of analyte(s) to the ion detection station 4 (parts IV and V).

Finally, following mass spectrometry, data evaluation (part VI), i.e. identification and possibly quantitation of each analyte of interest for each sample can be made.

In particular, the method describes herein is perfomed in a random-access mode, with a high-throughput MS set up, wherein several different analytes exhibiting different chemical properties have to be measured in a short amount of time.

High-throughput MS set up can mean that at least 10 samples per hour can be measured, preferably 100 samples /h or more than 100 samples per hour, e.g. 120 or 130 or 140 or 150 or 160 or 170 or 180 or 190 or 200 or 300 or 400 or 500 or 600 samples per hour.

Short amount of time can mean that a run for one sample is finished in 360s, preferably in excact 360s or more than 360 s.

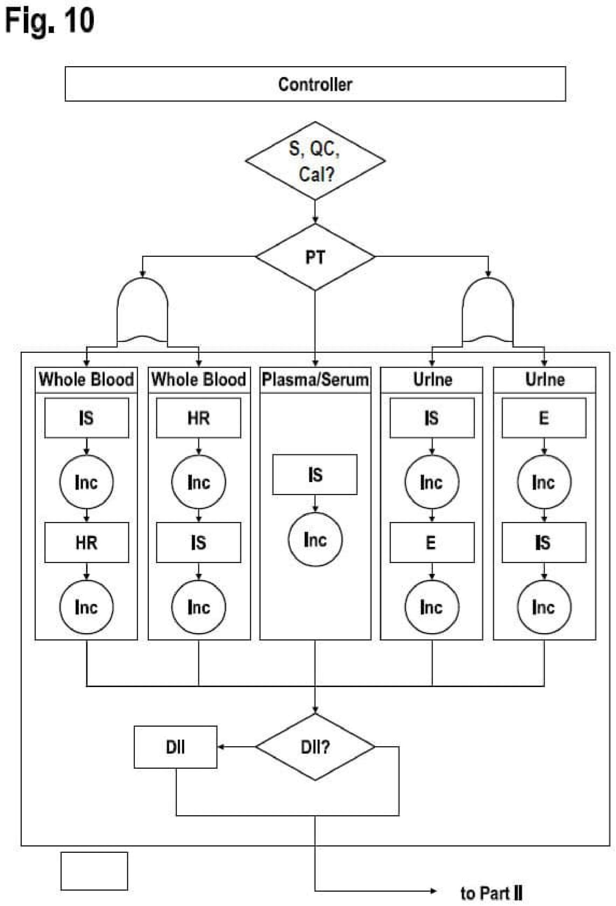

**Figure 10** is an examplified flow chart representing part I of the clinical diagnostic method of Figure 9 in more detail, and in particular the sample pre-treatment part. A controller first determines whether to assign a pre-defined workflow to a sample S or whether a quality control (QC) or a calibration (Cal) has to be carried out. A QC and/or a calibration workflow may follow at least some of the same steps as one of the sample workflows with the possible addition or deletion of some of the steps. For the purpose of this example only sample-type-dependent pre-treatment (PT) workflows are described.

For example, if the sample is a whole blood sample, it is assigned to one of two pre-defined sample PT workflows, both comprising the addition of an internal standard (IS) and a hemolysis reagent (HR) followed by a pre-defined incubation period (Inc), where the difference between the two workflows is the order in which the internal standard (IS) and a hemolysis reagent (HR) are added. An internal standard (IS) is typically a known amount of the same analyte(s) of interest that may be for example isotopically labeled. This allows relative comparison, and may enable unambiguous identification and quantification of the analyte(s) of interest present in the sample when the analyte(s) reach the mass spectrometer.

If the sample is a urine sample, it is assigned to one of other two pre-defined sample PT workflows, both comprising the addition of an internal standard (IS) and an enzymatic reagent (E) followed by a pre-defined incubation period (Inc), where the difference between the two workflows is the order in which the internal standard (IS) and a enzymatic reagent (HR) are added. An enzymatic reagent is typically a reagent used for glucuronide cleavage or protein cleavage or any pre-processing of analyte or matrix.

If the sample is plasma or serum it is assigned to another pre-defined PT workflow including only the addition of an internal standard (IS) followed by a pre-defined incubation time (Inc). Optionally, it may further include the addition of a lysis reagent (not shown).

All of the above sample-type-dependent PT workflows may comprise the addition of a dilution liquid (Dil). However, the addition of a dilution liquid (Dil) may be also specifically linked to any one or more of the above sample-type-dependent PT workflows.

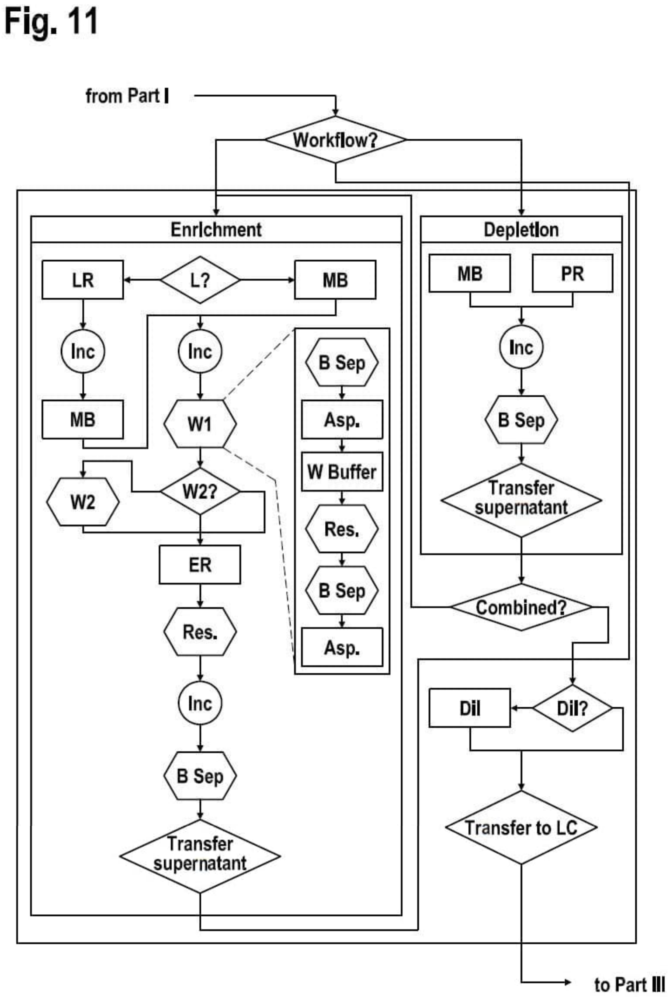

**Figure** 11 is an examplified flow chart representing Part II of the clinical diagnostic method of Figure 9 in more detail and is a continuation of the flow chart of Figure 10. In particular, the controller assigns each pre-treated sample to one of pre-defined analyte-dependent sample preparation workflows depending on the analyte(s) of interest in the pre-treated sample.

For example, a pre-treated sample can undergo an analyte enrichment workflow, a matrix depletion workflow or a matrix depletion workflow followed by an analyte enrichment workflow.

The analyte enrichment workflow comprises addition of magnetic beads (MB) carrying analyte-selective groups to the pre-treated sample followed by a pre-defined incubation period (Inc) for capturing the analyte(s) of interest, where the addition of the magnetic beads (MB) may include agitation or mixing. The addition of the magnetic beads (MB), depending on the analyte(s) of interest, may be preceded by addition of a lysis reagent (LR) followed by a pre-defined incubation period (Inc). A lysis reagent (LR) can be a reagent for lysis of erythrocytes or release from binding protein or release from unspecific binding. After incubation with the magnetic beads (MB) the workflow comprises a washing step (W1) and depending on the analyte(s) possibly one or more additional washing steps (W2). A washing step (W1, W2) comprises a series of steps including magnetic bead separation (B sep) by a magnetic bead handling unit comprising magnets or electromagnets, aspiration of liquid (Asp.), addition of a washing buffer (W. Buffer), resuspension of the magentic beads (Res.), another magnetic bead separation step (B Sep) and another aspiration of the liquid (Asp.). Moreover washing steps may differ in terms of type of solvent (water/organic/salt/pH), apart from volume and number or combination of washing cycles.

The last washing step (W1, W2) is followed by the addition of an elution reagent (ER) followed by resuspension (Res.) of the magnetic beads and a pre-defined incubation period (Inc.) for releasing the analyte(s) of interest from the magnetic beads. The bound-free magnetic beads are then separated (B Sep.) and the supernatant containing the analyte(s) of interest can be directly transferred to the LC station or after a dilution step by addition of a dilution liquid (Dil). Different elution procedures/reagents may also be used, by changing e.g. the type of solvents (water/organic/salt/pH) and volume.

The matrix depletion workflow comprises the addition of magnetic beads carrying matrix selective groups (MB) and a precipitation reagent (PR) to the pre-treated sample followed by a pre-defined incubation period (Inc.), for capturing the matrix components while leaving the analyte(s) of interest in the supernatant, followed by Bead separation (B Sep.) and transfer of the supernatant containing the analyte(s) of interest to LC station directly or after dilution (Dil).

Depending on the analyte(s) of interest, the supernatant derived from the matrix depletion workflow can undergo the analyte enrichment workflow following thereby a combined sample preparation workflow.

Parts III to VI can be continued after the examplified flow chart representing Parts I and II of the clinical diagnostic method of Figures 10 and 11. Additionally, a Part VII can be followed, which can be a computer unit for controlling the AC/DC and RF values, gas flows and/or temperatures of the certain units. The controlling is bidirectional controlled by readbacks of AC/DC and RF values, gas flows and/or temperatures of the certain units. The controlling can be triggerd within ms (milliseconds) up to hours depending on the run time of the dertain unit.

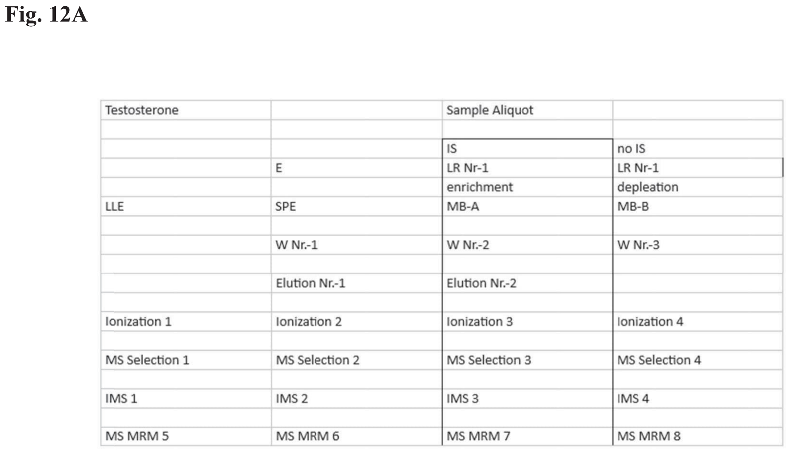

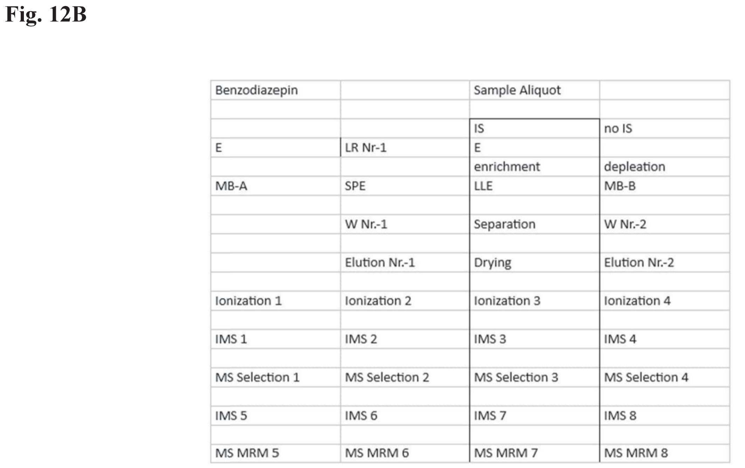

**Figures 12A** **and** **12B** provide two generic examples of analyte specific workflow paths that may be pre-defined in a master table or memory, each including a selection of options among generally selectable options (only most relevant options are indicated for simplicity). These general selectable options are for example the addition of an internal standard (IS) or no addition of IS (no IS); the addition of an enzymatic reagent (E) or one of two lysis reagents (LR #1, LR#2); the enrichment or depletion workflow; one type of magnetic beads (MB A, MB B, MB C, MB D) where different types of magnetic beads selective for groups of analytes (eg. polar, unpolar, charged, uncharged) or specific for selected analytes (specific binders); other types of sample pre enrichment like LLE (liquid-liquid extraction) or SPE (soplid phase extraction) are possible one of different pre-defined washing procedures (W Nr.-1, W Nr.-2, W Nr.-3, W Nr.-4) including different types of washing liquid (water/organic/salt/pH), volume, number or combination of washing cycles; type of Ionization (Ionization 1, Ionization 2, Ionization 3, Ionization 4, e.g. MAI, Paper Spray, MALDi, direct infusion ESI, nanao ESI, etc.); one of different pre-defined elution procedures (elution Nr.-1, elution Nr.-2, elution Nr.-3, elution Nr.-4), including different types of elution liquid (water/organic/salt/pH) or volume; type of ion mobility (with or without supporting gas in differnet AC/DC and RF and gas configurations); one of different pre-defined mass spectrometric acquisition procedures (settings for ionization, ion transfer, ion selection and fragmentation, detection and data acquisition) and shortly summarized as MS MRM 5, MS MRM 6, MS MRM 7, MS MRM 8, MS Selection by pre filtering e.g with ion trap, or multipole e.g. ion guide. The ion mobility is shortly summarized as IMS 1, IMS 2, IMS 3, IMS 4.

If the analyte of interest is for example testosterone, the testosterone-specific workflow includes the addition of an internal standard (IS), the addition of a lysis reagent (LR Nr.-1), an enrichment workflow, the addition of testosterone selective magnetic beads (MB A), one of the pre-defined washing procedure (W Nr.-2), one of the pre-defined elution procedure (elution Nr.-2), ionization 3 by nano-ESI, the use MS Selection Nr. 3 (Ion Trap) and Ion Mobility Nr. 3 (e.g. structures for lossless ion manipulations (SLIM)), and one of the pre-defined mass spectrometric acquisition procedures (MS MRM 7).

If the analytes of interest are for example benzodiazepines, the benzodiazepine-specific workflow includes the addition of an internal standard (IS), the addition of an enzymatic reagent (E), an enrichment workflow, the addition non mixable solvent to the sample media e.g. ethylacetate, liquid separation stept by pipetting, drying step on a surface for SALDI, SALDI ionization, MS Selection by a multipole, IMS by SLIM with supporting gas addition and one of the pre-defined mass spectrometric acquisition procedures (MS MRM 7).

Similarly, workflow paths may be pre-defined for any sample/analyte of interest or groups of analytes of interest.

Modifications and variations of the disclosed embodiments are certainly possible in light of the above description. It is therefore to be understood, that within the scope of the appended claims, the invention may be practiced otherwise than as specifically devised in the above examples.

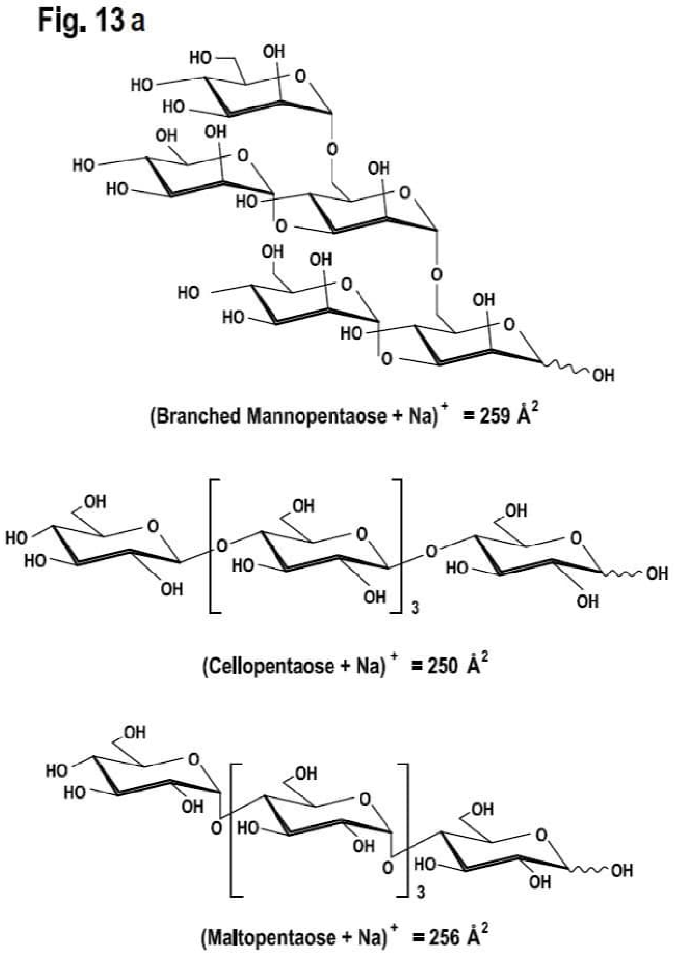

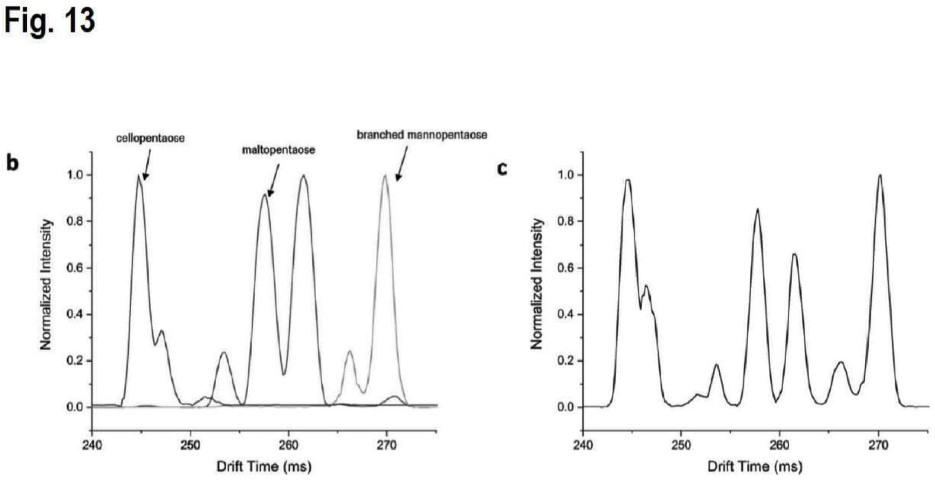

**Figure 13** shows in (a) structures of three isobaric pentasaccharides (branched mannopentaose, cellopentaose, and maltopentaose), used for analyses with corresponding CCS values (REF), in (b) SLIM-MS combined mobiligram of overlaid individual pentasaccharide mobiligrams, and in (c) a SLIM-MS mobiligram of the mixture of the three pentasaccharides according to the prior art. This comparative example shows that the separation of the three isobaric pentasaccharides can be done by SLIM-MS. In this certain case the analyte was injected into a nano-ESI device. However, in contrast to the present invention, this certain comparative application does not care about central elements of extraction and/or pre-anayltical steps, e.g. separation of polysugars from the matrix. Furthermore, the comparative applications outlined are only qualitatively and does not care about quantitative necessary steps e.g. usage and pipetting, e.g. of an isotopically labelled internal standard. For the example of polysugars the pseudomolecular ion [M+Na] has been chosen for separation with the SLIM device not carring about the fact that this pseudomolecular ion does not fragment enough within further MS/MS procedures. Therefore, a quantitative assessment using SLIM and a fragemtation unit is not foreseen in the outlined comparative examples. In contrast to that, the inventors surprisingly found that analytes of interest of biological samples can be qualitative and/or quantative measured by a clinical diagnostic system 100 according to the first aspect of the present invention. Preferably, the clinical diagnostic system 100 is automated and/or the the biological sample 9 is prepared in a random-excess mode.

This patent application claims the priority of the European patent application 20216371.3, wherein the content of this European patent application is hereby incorporated by references.

The following clauses are listed for better understanding the invention:
Clause 1. A clinical diagnostic system (100) comprising
   - a sample preparation station (1) for the automated preparation of biological samples (9) comprising at least one analyte of interest,
   - an ion generation station (2) for generating at least one ion or ions of the at least one analyte of interest,
   - at least one selectivity enhancer station (3) or more for enhancing the selectivity of the at least one analyte of interest, wherein the at least one selectivity enhancer station (3) separates ions based on the respective ion size comprises an ion mobility unit (31) for separation ions with respect to their ion mobility using electric and/or radio frequency fields (AC/DC and/or RF) applied to one or more electrodes,
   - an ion detection station (4) for detecting of at least one ion of the at least one analyte of interest, and
   - a data processing station (5) for processing and/or evaluation of at least one electronic signal received at least from the ion detection system.
Clause 2. The clinical diagnostic system (100) of clause 1, wherein the biological sample (9) is prepared in a random-excess mode and/or wherein the clinical diagnostic system (100) is automated.
Clause 3. The clinical diagnostic system (100) of clause 1 or 2, wherein the biological sample (9) is obtained from a patient sample, which is selected from a group consisting of serum, plasma, tissue, liquor, breath, sweat, sputum and whole blood sample from an individual, preferably wherein the individual is a mammal, preferably a human.
Clause 4. The clinical diagnostic system (100) of any of the proceeding clauses, wherein the at least one analyte of interest has a molar mass of smaller than m/z= 2000, preferably smaller than m/z =1000.
Clause 5. The clinical diagnostic system (100) of any of the proceeding clauses, wherein the sample preparation station (1) is for removing or at least reducing interfering matrix components in the biological sample (9) and/or enriching analytes of interest in the biological sample (9) and comprises at least one of the following units or combinations thereof: sample delivery unit, sample pre-treatment unit, sample analytical unit.
Clause 6. The clinical diagnostic system (100) of any of the proceeding clauses, wherein the sample delivery unit comprises sample collection and preparation and/or transportation, and/or
   wherein the sample pre-treatment unit comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit and/or
   wherein the sample analytical unit comprises at least one unit or more than one units selected from the group: a solid-liquid support analyte enrichment unit, solid-liquid support matrix depletion unit, gaseous sample enrichment or matrix separation unit, solid sample on solid support unit.
Clause 7. The clinical diagnostic system (100) of any of the proceeding clauses, wherein the sample preparation station (1) is partially or fully automated.
Clause 8. The clinical diagnostic system (100) of any of the proceeding clauses, wherein the ion generation station (2) comprises an unsteady analyte ion supply unit (21) and/or a steady analyte ion supply unit (22), wherein the unsteady analyte ion supply unit (21) is selected from the group consisting of laser desorption ionzation (LDI, 211), dielectric barrier liquid flow (212), surface plasma ionization (213) and combinations thereof, wherein the steady analyte ion supply unit (22) is selected from the group consisting of flow injection (221), paper spray (222), electron ionization (EI, 223), matrix assisted ionization (MAI, 223), chemical ionization (CI, 225), e.g. proton-transfer-reaction (PTR), radioactive supported ionization (226), e.g. using 63Ni and combinations thereof.
Clause 9. The clinical diagnostic system (100) of any of the proceeding clauses, wherein the selectivity enhancer station (3) comprises the ion mobility unit (31) and at least one unit selected from the following group: ion transportation unit (32), ion fragmentation unit (33), wherein the ion transportation unit (32) is selected from the group consisting of ion funnels (321), step wave (322), S-lens (323), ion mirror (324), ion fight tube by differential potential (325), ion trap (326), multipole (327), e.g. quadrupole, non-contact ion transport system (328), e.g. direct source coupling or heated capillary, and magnetic sector transport (329), wherein the ion fragmentation unit (33) is selected from the group consisting of collision-induced dissociation (CID, 331), electron-capture dissociation (ECD, 332), electron-transfer dissociation (ETD, 333), photon dissociation (334), ion trap (335), orbitrap (336), time-of-flight (TOF, 337), magnetic sector (338), e.g. double focussion sector field (DFS) and combinations thereof.
Clause 10. The clinical diagnostic system (100) of any of the proceeding clauses, wherein the at least one analyte of interest is present in the said clinical diagnostic system (100) or in the mass spectrometer or in the ion mobility unit (31) of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.
Clause 11. Use of the clinical diagnostic system (100) of any one of clauses 1 to 10 for determining the presence or the level of the at least one analyte of interest in the biological sample (9).
Clause 12. A method for determining the presence or level of at least one analyte of interest in a biological sample (9) comprising:
   A) Preparation of the biological sample (9), preferably automated preparation of the biological sample (9) comprising at least one analyte of interest,
   B) Ion generation from the at least one analyte of interest,
   C) Separation the ion or ions via their ion mobility using an electric field applied to one or more electrodes,
   D) Detection and determining of at least one ion of the at least one analyte of interest using mass spectrometry.
Clause 13. The method of clause 12, wherein the internal standard is added, preferably in step (A), wherein the internal standard is preferably isotopically labelled.
Clause 14. The method of clauses 12 or 13, wherein the said method is performed in a clinical diagnostic system (100) according to any one of clauses 1 to 10.
Clause 15. A kit suitable to perform a method of clause 12 or 13 or 14 comprising
   - reagents, preferably for performing the pre-treatment,
   - magnetic, paramagnetic or supraparamagnetic beads for performing the enrichment, and
   - optionally an internal standard, which is preferably isotopically labelled.
Clause 16. Use of the kit of clause 15 in a clinical diagnostic system (100) of any one of clauses 1 to 10 and/or in a method of any one of clauses 12 to 14.

### List of References

100 - clinical diagnostic system
1 - sample preparation station
   11 - sample delivery unit
   12 - sample pre-treatment unit
      121 - magnetic bead handling unit
      122 - internal standard handling unit
      123 - supporting compound handling unit
      124 - pipetting unit
      125 - fluid transport unit
      126 - reaction container transporting mechanism unit
      127 -enrichment handling unit
      128 - solvent evaporation unit
   13 - sample analytical unit
      131 - solid-liquid support analyte enrichment unit
      132 - solid-liquid support matrix depletion unit
      133 - gaseous sample enrichment or matrix separation unit
      134 - solid sample on solid support unit
2- ion generation station
   21 - unsteady analyte ion supply unit
      211 - laser desorption ionzation (LDI)
      212 - dielectric barrier liquid flow
      213 - surface plasma ionization
   22 - steady analyte ion supply unit
      221 - flow injection (preferably flow injection using ESI or APCI or Nano Spray)
      222 - paper spray
      223 - electron ionization (EI)
      224 - matrix assisted ionization (MAI)
      225 - chemical ionization (CI), e.g. proton-transfer-reaction (PTR)
      226 - radioactive supported ionization, e.g. using ⁶³Ni.
3 - at least one selectivity enhancer station
   31 - ion mobility unit
   32 - ion transportation unit
      321 - ion funnels
      322 - step wave
      323 - S-lens
      324 - ion mirror
      325 - ion fight tube by differential potential
      326 - ion trap
      327 - multipole, e.g. quadrupole
      328 - non-contact ion transport system (e.g. heated capillary)
      329 - magnetic sector transport
   33 - at least one ion fragmentation unit
      331 - collision-induced dissociation (CID)
      332 - electron-capture dissociation (ECD)
      333 - electron-transfer dissociation (ETD)
      334 - photon dissociation
      335 - ion trap
      336 - orbitrap
      337 - time-of-flight (TOF)
      338 - magnetic sector, e.g. double focussion sector field (DFS)
4 - ion detection station
   41 - faraday cup
   42 - microchannel plate detector (MCP)
   43 - photomultiplier
   45 - orbitrap
   46 - electron multiplier
   47 - photographic plate
5 - data processing station
6 - coupling between the stations
7 - coupling between the units
8 - coupling to the adjacent stations and/or units
9 - sample or biological sample or samples or biological samples
N - number of selectivity enhancer stations, where N ≥ 1, e.g. 1 or 2 or 3 or 4 or 5 or 6
M - number of ion fragmentation unit, where M ≥ 0
Z - number of ion fragmentation unit, where M ≥ 0

## Claims

1. A clinical diagnostic system (100) comprising
- a sample preparation station (1) for the automated preparation of biological samples (9) comprising at least one analyte of interest, wherein the sample preparation station comprises a sample pre-treatment unit, which comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit;
- an ion generation station (2) for generating at least one ion or ions of the at least one analyte of interest;
- at least one selectivity enhancer station (3) or more for enhancing the selectivity of the at least one analyte of interest, wherein the at least one selectivity enhancer station (3) separates ions based on the respective ion size comprises an ion mobility unit (31) for separation ions with respect to their ion mobility using electric and/or radio frequency fields (AC/DC and/or RF) applied to one or more electrodes;
- an ion detection station (4) for detecting of at least one ion of the at least one analyte of interest; and
- a data processing station (5) for processing and/or evaluation of at least one electronic signal received at least from the ion detection system.

2. The clinical diagnostic system (100) of claim 1, wherein the biological sample (9) is prepared in a random-excess mode and/or wherein the clinical diagnostic system (100) is automated.

3. The clinical diagnostic system (100) of claim 1 or 2, wherein the biological sample (9) is obtained from a patient sample, which is selected from a group consisting of serum, plasma, tissue, liquor, breath, sweat, sputum and whole blood sample from an individual, preferably wherein the individual is a mammal, preferably a human.

4. The clinical diagnostic system (100) of any of the proceeding claims, wherein the at least one analyte of interest has a molar mass of smaller than m/z= 2000, preferably smaller than m/z =1000.

5. The clinical diagnostic system (100) of any of the proceeding claims, wherein the sample preparation station (1) is for removing or at least reducing interfering matrix components in the biological sample (9) and/or enriching analytes of interest in the biological sample (9) and comprises at least one of the following units or combinations thereof: sample delivery unit, sample analytical unit.

6. The clinical diagnostic system (100) of claim 5, wherein the sample delivery unit comprises sample collection and preparation and/or transportation, and/or
wherein the sample analytical unit comprises at least one unit or more than one units selected from the group: a solid-liquid support analyte enrichment unit, solid-liquid support matrix depletion unit, gaseous sample enrichment or matrix separation unit, solid sample on solid support unit.

7. The clinical diagnostic system (100) of any of the proceeding claims, wherein the sample preparation station (1) is partially or fully automated.

8. The clinical diagnostic system (100) of any of the proceeding claims, wherein the ion generation station (2) comprises an unsteady analyte ion supply unit (21) and/or a steady analyte ion supply unit (22), wherein the unsteady analyte ion supply unit (21) is selected from the group consisting of laser desorption ionzation (LDI, 211), dielectric barrier liquid flow (212), surface plasma ionization (213) and combinations thereof, wherein the steady analyte ion supply unit (22) is selected from the group consisting of flow injection (221), paper spray (222), electron ionization (EI, 223), matrix assisted ionization (MAI, 223), chemical ionization (CI, 225), e.g. proton-transfer-reaction (PTR), radioactive supported ionization (226), e.g. using ⁶³Ni and combinations thereof.

9. The clinical diagnostic system (100) of any of the proceeding claims, wherein the selectivity enhancer station (3) comprises the ion mobility unit (31) and at least one unit selected from the following group: ion transportation unit (32), ion fragmentation unit (33), wherein the ion transportation unit (32) is selected from the group consisting of ion funnels (321), step wave (322), S-lens (323), ion mirror (324), ion fight tube by differential potential (325), ion trap (326), multipole (327), e.g. quadrupole, non-contact ion transport system (328), e.g. direct source coupling or heated capillary, and magnetic sector transport (329), wherein the ion fragmentation unit (33) is selected from the group consisting of collision-induced dissociation (CID, 331), electron-capture dissociation (ECD, 332), electron-transfer dissociation (ETD, 333), photon dissociation (334), ion trap (335), orbitrap (336), time-of-flight (TOF, 337), magnetic sector (338), e.g. double focussion sector field (DFS) and combinations thereof.

10. The clinical diagnostic system (100) of any of the proceeding claims, wherein the at least one analyte of interest is present in the said clinical diagnostic system (100) or in the mass spectrometer or in the ion mobility unit (31) of less than 10 seconds, preferably less than 9 seconds or less than 8 seconds or less than 7 seconds or less than 6 seconds or less than 5 seconds or less than 4 seconds or less than 3 seconds or less than 2 seconds or less than 1 second.

11. Use of the clinical diagnostic system (100) of any one of claims 1 to 10 for determining the presence or the level of the at least one analyte of interest in the biological sample (9).

12. A method for determining the presence or level of at least one analyte of interest in a biological sample (9) comprising:
A) Preparation of the biological sample (9), preferably automated preparation of the biological sample (9) comprising at least one analyte of interest, using a sample pre-treatment unit, which comprises at least one unit or more than one units selected from the following group: magnetic bead handling unit, internal standard handling unit, supporting compound handling unit, pipetting unit, fluid transport unit, reaction container transporting mechanism unit, enrichment handling unit, solvent evaporation unit, derivatization unit
B) Ion generation from the at least one analyte of interest,
C) Separation the ion or ions via their ion mobility using an electric field applied to one or more electrodes,
D) Detection and determining of at least one ion of the at least one analyte of interest using mass spectrometry.

13. The method of claim 12, wherein the internal standard is added, preferably in step (A), wherein the internal standard is preferably isotopically labelled.

14. A kit suitable to perform a method of claim 12 or 13 comprising
- reagents, preferably for performing the pre-treatment,
- magnetic, paramagnetic or supraparamagnetic beads for performing the enrichment, and
- optionally an internal standard, which is preferably isotopically labelled.

15. Use of the kit of claim 14 in a clinical diagnostic system (100) of any one of claims 1 to 10 and/or in a method of any one of claims 12 to 13.
